# EUROPEAN PATENT SPECIFICATION

(11) **EP 3 378 940 B1**
(45) Date of publication and mention of the grant of the patent: **03.06.2020**
(21) Application number: 16860244.9
(22) Date of filing: 27.10.2016
(51) Int. Cl.: C12N 15/70, C12N 15/81, C12P 7/18, C12N 9/04, C12N 9/02, C07H 21/04

(54) **METHOD FOR PRODUCING HEAVY CHAIN DIOL**
VERFAHREN ZUR HERSTELLUNG VON SCHWERKETTIGEM DIOL
PROCÉDÉ DE PRODUCTION D'UN DIOL À CHAÎNE LOURDE

(30) Priority: 27.10.2015 KR 20150149251
(43) Date of publication of application: 26.09.2018
(73) Proprietor: Korea Research Institute of Bioscience and Biotechnology, Daejeon 34141 (KR)
(72) Inventor: AHN, Jung Oh, Daejeon 34141 (KR); LEE, Hong Weon, Daejeon 34141 (KR); JANG, Min Jeong, Daejeon 34141 (KR); KIM, Chun Sug, Daejeon 34141 (KR); PARK, Gyu Yeon, Daejeon 34141 (KR)
(74) Representative: Cabinet Laurent & Charras
(86) International application number: PCT/KR2016/012170
(87) International publication number: WO 2017/074061

(56) References cited:
- WO-A1-2014/201474
- DE-A1-102014 220 186
- KR-A- 20140 142 518
- KR-A- 20150 039 055
- KR-B1- 101 145 405
- US-B2- 8 530 206
- PARK ET AL.: 'Metabolic Engineering of Escherichia Coli for the Production of Medium-chain-length Polyhydroxyalkanoates Rich in Specific Monomers' FEMS MICROBIOLOGY LETTERS vol. 214, 2002, pages 217 - 222, XP055227754

## Description

### [Technical Field]

The present invention relates to a method for producing a medium chain diol, and more particularly, to a method for producing a medium chain diol from a fatty acid-derived alcohol or alkane by culturing a recombinant microorganism from which the fatty alcohol dehydrogenase, the fatty alcohol oxidase and the fatty aldehyde dehydrogenase genes in the ω-oxidative metabolism pathway are deleted, and the β-oxidative metabolism pathway-related genes are also deleted.

### [Background Art]

Bioplatform compounds are produced through biological or chemical conversion on the basis of biomass-derived raw materials, and have been used for synthesis of polymeric monomers, new materials, and the like.

Among the bioplatform compounds, a medium chain diol is a material used as a monomer for polyesters. In this case, the polyesters have been used for various applications including fibers, films, and combinations thereof due to their excellent properties. For example, the polyethylene terephthalate obtained through the polycondensation of terephthalic acid with ethylene glycol has been used for many applications due to excellent physical properties such as mechanical strength, chemical properties, and the like, and has been mass-produced as a synthetic fiber most suitable for clothing all over the world. Also, the market for polytrimethylene terephthalate prepared using 1,3-propanediol and terephthalic acid as raw materials tends to increase with the recent development of inexpensive methods of synthesizing 1,3-propanediol. Thus, the polytrimethylene terephthalate is expected to be employed for clothing applications requiring the soft texture because it has polymeric characteristics such as excellent elastic recovery percentage of elongation and low Young's modulus. In addition, the biomass resource-derived polyesters have come into the spotlight for fear of the rising price and depletion of petroleum resources.

Production of medium chain diols may be carried out using biological methods through chemical synthesis or microbial fermentation. In this case, the use of such biological methods requires the development of novel strains and the optimization of fermentation processes using metabolic engineering technology.

In the prior art, a microorganism which harbors a β-oxidative metabolism pathway as well as an ω-oxidative metabolism pathway may be used as the strain capable of producing a medium chain diol. For example, it is known that the strains such as *Klebsiella oxytoca, Klebsiella pneumoniae, Aerobacter aerogenes,* recombinant *Saccharomyces cerevisiae*, and the like may produce 2,3-butanediol with high yield and high productivity (Korean Patent Publication Nos. 10-2012-0107021, 10-2012-0128776, and 10-2015-0068581). However, because some of these microorganisms are classified into pathogenic microorganisms, they have limitations in terms of safety and industrialization. Also, because the medium chain diols correspond to intermediate products in the ω-oxidative metabolism pathway, the medium chain diols have a problem in that the medium chain diols may not be produced with high yield when they are produced using the microorganisms.

DE 10 2014 220186 discloses a recombinant mutant for producing ω-hydroxy fatty acids and dicarboxylic acids and WO 2014/201474 relates to a method of producing ω-hydroxylated fatty acid derivatives.

### [Disclosure]

### [Technical Problem]

Therefore, it is an object of the present invention to provide a recombinant microorganism from which the fatty alcohol dehydrogenase, the fatty alcohol oxidase-related and the fatty aldehyde dehydrogenase genes in the ω-oxidative metabolism pathway are deleted, and the β-oxidative metabolism pathway-related genes are also deleted, and a method of producing a medium chain diol from a fatty acid-derived alcohol or alkane by culturing the recombinant microorganism.

### [Technical Solution]

To solve the above problems, according to an aspect of the present invention, there is provided a recombinant microorganism from which the fatty alcohol dehydrogenase, the fatty alcohol oxidase and the fatty aldehyde dehydrogenase genes in the ω-oxidative metabolism pathway are deleted, and the β-oxidative metabolism pathway-related genes are also deleted.

According to an embodiment of the present invention, the fatty alcohol dehydrogenase gene, the fatty alcohol oxidase gene, the fatty aldehyde dehydrogenase gene, and the β-oxidative metabolism pathway-related genes may be deleted from all homologous genes present in the microorganism. According to another embodiment of the present invention, the fatty alcohol dehydrogenase gene, the fatty alcohol oxidase gene, the fatty aldehyde dehydrogenase gene, and the β-oxidative metabolism pathway-related genes may be deleted from some of the homologous genes present in the corresponding microorganism.

According to the present disclosure, the fatty alcohol dehydrogenase gene may be selected from the group consisting of ADH1, ADH2, ADH3, ADH4, ADH5, ADH6, ADH7, ADH8, and FADH genes, but the present disclosure is not limited thereto. According to the present disclosure, the fatty alcohol oxidase gene may be an FAO gene, but the present disclosure is not limited thereto. According the present disclosure, the fatty aldehyde dehydrogenase gene may be a gene selected from the group consisting of FALDH1, FALDH2, FALDH3, and FALDH4 genes, but the present disclosure is not limited thereto.

According to an embodiment of the present invention, the β-oxidative metabolism pathway-related genes is an acyl-CoA oxidase gene. According to the present disclosure, the acyl-CoA oxidase gene may be selected from the group consisting of ACO1, ACO2, ACO3, ACO4, ACO5, and ACO6 genes, but the present disclosure is not limited thereto.

According to an embodiment of the present invention, the microorganism is a yeast or *Escherichia coli.*

According to preferred embodiments of the present invention, the yeast is selected from the group of the yeast consisting of *Yarrowia* sp., *Saccharomyces* sp., *Pichia* sp., and *Candida* sp..

According to other preferred embodiments of the present invention, the *Yarrowia* sp. yeast may be *Yarrowia lipolytica.*

According to another aspect of the present invention, there is provided a method for producing a medium chain diol, which comprises (1) preparing a recombinant microorganism from which the fatty alcohol dehydrogenase, the fatty alcohol oxidase and the fatty aldehyde dehydrogenase genes in the ω-oxidative metabolism pathway are deleted, and the β-oxidative metabolism pathway-related genes are also deleted; and (2) treating the recombinant microorganism with a substrate to culture the recombinant microorganism.

According to an embodiment of the present invention, the substrate is selected from the group consisting of a fatty acid-derived alcohol and alkane.

According to preferred embodiments of the present invention, each of the fatty acid-derived alcohol and alkane and the medium chain diol has 5 to 30 carbon atoms, preferably 6 to 20 carbon atoms, and more preferably 8 to 16 carbon atoms.

According to the present disclosure, the alkane may be dodecane, but the present disclosure is not limited thereto.

According to still other preferred embodiments of the present invention, the medium chain diol may be 1,12-dodecanediol, but the present disclosure is not limited thereto.

### [Advantageous Effects]

Because the fatty alcohol dehydrogenase, the fatty alcohol oxidase genes, the fatty aldehyde dehydrogenase genes in the ω-oxidative metabolism pathway are deleted, and the β-oxidative metabolism pathway-related genes are also deleted from a recombinant microorganism according to the present invention, the recombinant microorganism can produce medium chain diols with high yield by preventing further oxidation and β-oxidative metabolism of fatty alcohols.

### [Description of Drawings]

FIG. 1 is a diagram showing types of products and related enzymes associated with ω-oxidative and β-oxidative metabolism reactions.
FIG. 2 is a diagram schematically showing a process of preparing a recombinant microorganism of the present invention from which fatty alcohol dehydrogenase, fatty alcohol oxidase and fatty aldehyde dehydrogenase genes associated with ω-oxidation and an acyl-CoA oxidase gene associated with β-oxidation are deleted.
FIG. 3 is a diagram schematically showing a vector containing an ura3 gene to be used as a selective marker for gene knockout to modify a strain, and a pop-out region for deleting the ura3 gene after insertion of a knock-out cassette.
FIG. 4 is a schematic diagram showing a process of constructing a knock-out cassette used to prepare a transformant microorganism according to the present invention.
FIG. 5 is a graph illustrating types of knock-out genes in the transformant microorganism according to the present invention.
FIG. 6 is a graph illustrating an amount of a medium chain diol produced from the alkane substrate, using the transformant microorganism according to the present invention.
FIG. 7 is a graph illustrating an amount of the medium chain diol produced from the alkane substrate, when an Y4-20 strain of the present invention is cultured in a flask.
FIG. 8 shows the GC/MS data showing that the medium chain diol is produced the alkane substrate in the Y4-20 strain according to the present invention.

### [Best Mode]

The present invention provides a recombinant microorganism from which the fatty alcohol dehydrogenase, the fatty alcohol oxidase and the fatty aldehyde dehydrogenase genes in the ω-oxidative metabolism pathway are deleted, and the β-oxidative metabolism pathway-related genes are also deleted.

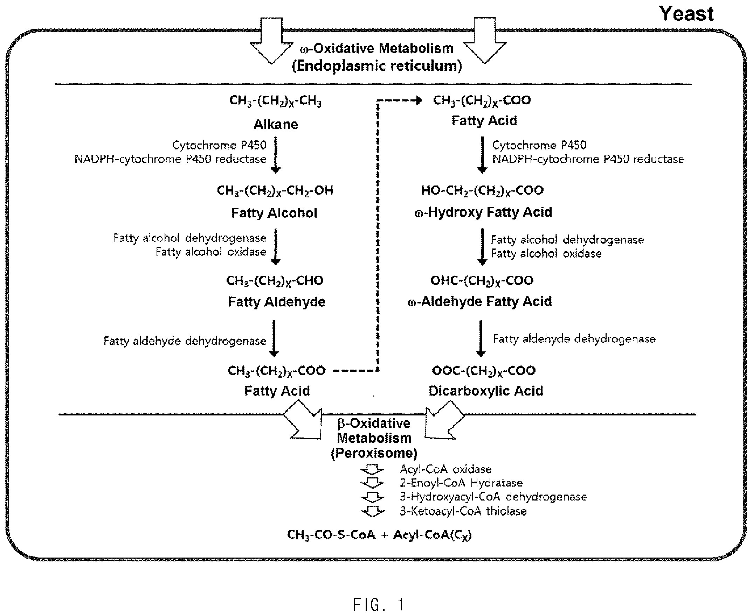

In the present invention, the term "ω-oxidation" refers to a metabolic process in which the terminal methyl group of a fatty acid is oxidized to form dicarboxylic acid, and the term "β-oxidation" refers to a metabolic process in which a carbon atom at the β-position in a carboxyl group is oxidized to release acetyl-CoA, whereby fatty acids are gradually decomposed to form fatty acids whose number of carbon atoms is reduced by two. The concept of the ω- and β-oxidations and the enzymes involved in such metabolic processes are widely known to persons having ordinary skill in the field of biochemistry. For example, when a fatty acid is used as the substrate for ω-oxidation, an ω-hydroxy fatty acid is first produced by means of an action of cytochrome P450 and an NADPH-cytochrome P450 reductase. Then, the ω-hydroxy fatty acid is converted into ω-aldehyde fatty acid by an action of a fatty alcohol dehydrogenase and a fatty alcohol oxidase, and the ω-aldehyde fatty acid is converted into dicarboxylic acid by an action of a fatty aldehyde dehydrogenase. Also, for the β-oxidation, a fatty acid whose number of carbon atoms is reduced by two is produced by an acyl-CoA oxidase (see FIG. 1).

According to an embodiment of the present invention, the fatty alcohol dehydrogenase gene, the fatty alcohol oxidase gene, the fatty aldehyde dehydrogenase gene, and the β-oxidative metabolism pathway-related genes are deleted from all homologous genes present in the corresponding microorganism, but a recombinant microorganism from which some of these genes are deleted may also be applied to the present invention, when necessary.

According to an embodiment of the present invention, the fatty alcohol dehydrogenase gene is selected from the group consisting of ADH1, ADH2, ADH3, ADH4, ADH5, ADH6, ADH7, ADH8, and FADH genes, and preferably each of the ADH1, ADH2, ADH3, ADH4, ADH5, ADH6, ADH7, ADH8, and FADH genes comprises base sequences set forth in SEQ ID NOs: 1 to 9, respectively.

According to another embodiment of the present invention, the fatty alcohol oxidase gene is an FAO gene and preferably the FAO gene comprises a base sequence set forth in SEQ ID NO: 10.

According to still another embodiment of the present invention, the fatty aldehyde dehydrogenase gene is selected from the group consisting of FALDH1, FALDH2, FALDH3, and FALDH4 genes and preferably each of the FALDH1, FALDH2, FALDH3, and FALDH4 genes comprises base sequences set forth in SEQ ID NOs: 11 to 14, respectively.

According to an embodiment of the present invention, the β-oxidative metabolism pathway-related genes are preferably deleted from all homologous genes present in the corresponding microorganism, but a recombinant microorganism from which some of these genes are deleted may also be applied to the present invention, when necessary.

According to an embodiment of the present invention, the β-oxidative metabolism pathway-related genes is an acyl-CoA oxidase gene.

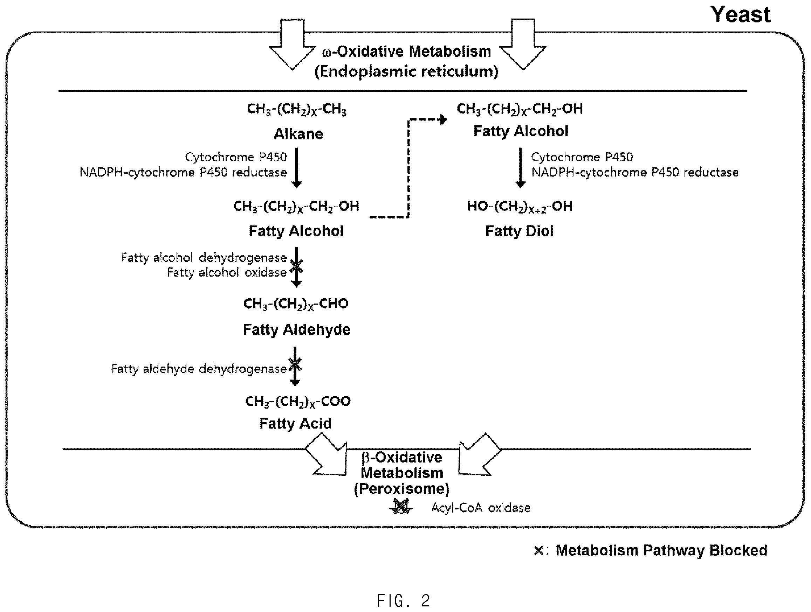

According to an embodiment of the present invention, the acyl-CoA oxidase gene is selected from the group consisting of ACO1, ACO2, ACO3, ACO4, ACO5, and AC06 genes, but the present disclosure is not limited thereto (see FIG. 2). According to other preferred embodiments of the present invention, the ACO1, ACO2, ACO3, ACO4, AC05, and AC06 genes comprise base sequences set forth in SEQ ID NOs: 15 to 20, respectively.

In the present invention, the recombinant microorganism from which the genes selected from the fatty alcohol dehydrogenase gene, the fatty alcohol oxidase gene, the fatty aldehyde dehydrogenase gene, and the acyl-CoA oxidase gene are deleted may be prepared using conventional genetic recombinant technology known in the related art. In the present invention, the term "deletion" is used as a meaning generally encompassing a physical deletion of part or all of the corresponding gene, and also encompassing a situation in which a protein is not expressed from mRNA transcribed from the corresponding gene and a situation in which a protein expressed from the corresponding gene does not function. Examples of the genetic recombinant technology that may be used herein may include methods such as transformation, transduction, transfection, microinjection, electroporation, and the like, but the present disclosure is not limited thereto.

In the present disclosure, any microorganisms having both ω-oxidative and β-oxidative metabolism processes may be used without limitation. For example, according to a preferred embodiment of the present invention, eukaryotes including a yeast and prokaryotes including *Escherichia coli* may be used. According to an embodiment of the present invention, the yeast is preferably used as the microorganism. In this case, yeasts such as *Yarrowia* sp., *Saccharomyces* sp., *Pichia* sp., *Candida* sp., and the like may be used as the yeast without limitation. Among theses, *Yarrowia lipolytica, Candida tropicalis*, *Candida infanticola*, *Saccharomyces cerevisiae, Pichia alcoholophia,* or *Candida mycoderma* is preferably used. *Yarrowia lipolytica* is more preferably used.

As described above, in the case of the microorganism from which the fatty alcohol dehydrogenase, the fatty alcohol oxidase and the fatty aldehyde dehydrogenase genes in the ω-oxidative metabolism pathway are deleted, and the β-oxidative metabolism pathway-related genes are deleted, when the alkane is supplied as the substrate, one of both terminals of the alkane is oxidized by an action of cytochrome P450 and an NADPH-cytochrome P450 reductase to form a primary alcohol. However, because the fatty alcohol dehydrogenase gene and the fatty alcohol oxidase gene are deleted, no further oxidation occurs anymore. Also, the primary alcohol thus formed is again used as a substrate so that the other terminal of the primary alcohol is oxidized by an action of the cytochrome P450 and the NADPH-cytochrome P450 reductase to form a diol as a secondary alcohol. When the alkane is used as the substrate as described above, the diol is formed through a two-step oxidation reaction, whereas the diol is formed through a one-step oxidation reaction when the alcohol other than the alkane is used as the substrate.

Also, the present invention provides a method for producing a medium chain diol, which comprises:
(1) preparing a recombinant microorganism from which the fatty alcohol dehydrogenase, the fatty alcohol oxidase and the fatty aldehyde dehydrogenase genes in the ω-oxidative metabolism pathway are deleted, and the β-oxidative metabolism pathway-related genes are also deleted; and
(2) treating the recombinant microorganism with a substrate to culture the recombinant microorganism.

In the present invention, the recombinant microorganism, from which the fatty alcohol dehydrogenase gene, the fatty alcohol oxidase gene and the fatty aldehyde dehydrogenase gene in the ω-oxidative metabolism pathway are deleted, and the β-oxidative metabolism pathway-related genes are also deleted, may be used to produce medium chain diols with high yield by preventing additional oxidation and β-oxidative metabolism of fatty alcohols. The fatty alcohol dehydrogenase gene, the fatty alcohol oxidase gene, the fatty aldehyde dehydrogenase gene, and the β-oxidative metabolism pathway-related genes are preferably deleted from all homologous genes present in the corresponding microorganism, but a recombinant microorganism from which some of these genes are deleted may also be applied to the present invention, when necessary.

In the present disclosure, any microorganisms having both ω-oxidative and β-oxidative metabolism processes may be used without limitation. For example, eukaryotes including a yeast and prokaryotes including *Escherichia coli* may be used. According to an embodiment of the present invention, the yeast is preferably used as the microorganism. In this case, yeasts such as *Yarrowia* sp., *Saccharomyces* sp., *Pichia* sp., *Candida* sp., and the like may be used as the yeast without limitation. Among theses, *Yarrowia lipolytica, Saccharomyces cerevisiae, Candida tropicalis*, *Candida infanticola*, *Pichia alcoholophia*, or *Candida mycoderma* is preferably used. *Yarrowia lipolytica* is more preferably used.

In the present invention, the recombinant microorganism from which the genes selected from the fatty alcohol dehydrogenase gene, the fatty alcohol oxidase gene, the fatty aldehyde dehydrogenase gene, and the acyl-CoA oxidase gene are deleted may be prepared using conventional genetic recombinant technology known in the related art. In the present invention, the term "deletion" is used as a meaning generally encompassing a physical deletion of part or all of the corresponding gene, and also encompassing a situation in which a protein is not expressed from mRNA transcribed from the corresponding gene and a situation in which a protein expressed from the corresponding gene does not function.

In the present invention, the term "diol" generally refers to a compound that contains two hydroxyl groups (-OH groups), and the term "medium chain diol" is used as a meaning encompassing all diol compounds having 5 to 30 carbon atoms, preferably 6 to 20 carbon atoms, and more preferably 8 to 16 carbon atoms.

In the present invention, the substrate of step (2) may be selected from the group consisting of a fatty acid-derived alcohol and alkane. According to an embodiment of the present invention, alcohols having 5 to 30 carbon atoms, preferably 6 to 20 carbon atoms, and more preferably 8 to 16 carbon atoms may be used as the fatty acid-derived alcohol. According to another embodiment of the present invention, alkanes having 5 to 30 carbon atoms, preferably 6 to 20 carbon atoms, and more preferably 8 to 16 carbon atoms may be used as the alkane. According to preferred embodiments of the present disclosure, the alkane may be dodecane, but the present disclosure is not limited thereto. According to other preferred embodiments of the present invention, the medium chain diol may be 1,12-dodecanediol, but the present disclosure is not limited thereto.

### [Mode for Invention]

Hereinafter, the present invention will be described in further detail with reference to examples thereof.

However, it should be understood that the following examples are just preferred examples for the purpose of illustration only and is not intended to limit or define the scope of the invention.

### Example 1: Construction of knock-out cassette

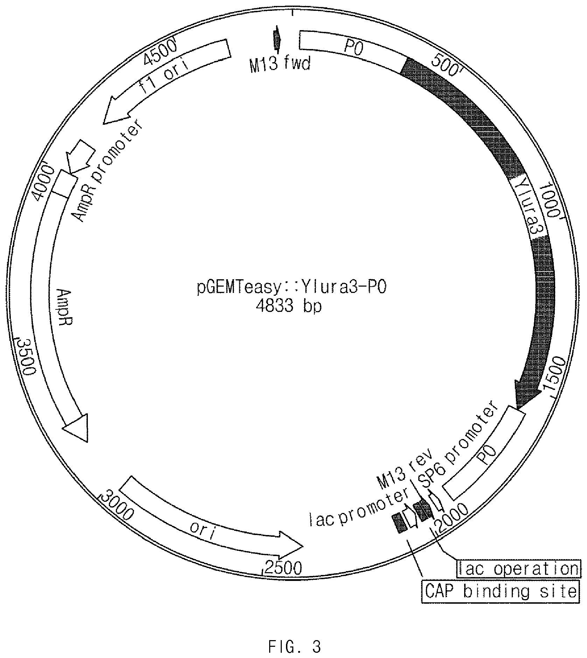

A vector containing an ura3 gene to be used as a selective marker for gene knockout to modify a strain, and a pop-out region for deleting the ura3 gene after insertion of a knock-out cassette was constructed (FIG. 3). A *Yarrowia*-derived gene was used as the ura3 gene, and the pop-out region used to modify a strain had a total of four sequences, and was referenced from two genes. Here, a *Bacillus*-derived glutamate-producing gene was used as one of the genes, and a gene associated with a *Salmonella-* or cloning vector pHUKH-derived His operon was used as the other one. The primers used to construct the pop-out vectors, and sequences thereof are listed in the following Table 1.

**[Table 1]**

| Pop-out Vectors | | | |
|---|---|---|---|
| Names | | **Base Sequences** | **SEQ ID NOs** |
| **HisG1** | BglII F | aattgggcccagatctcagaccggttcagacaggat | 22 |
| | EcoRI R | tctctgggcggaattcggaggtgcggatatgaggta | 23 |
| | NotI F | tgTTTCTCGgcggccgccagaccggttcagacaggat | 24 |
| | BamHI R | TCCAACGCGTGGATCCggaggtgcggatatgaggta | 25 |
| **HisG2** | BglII F | aattgggcccagatctaacgctacctcgaccagaaa | 26 |
| | EcoRI R | tctctgggcggaattctcttctcgatcggcagtacc | 27 |
| | NotI F | tgTTTCTCGgcggccgcaacgctacctcgaccagaaa | 28 |
| | BamHI R | TCCAACGCGTGGATCCtcttctcgatcggcagtacc | 29 |
| **glt2** | BglII F | aattgggcccagatctTCAGAACTTGCGCCGATAAA | 30 |
| | EcoRI R | tctctgggcggaattcCTTTGCCAGCTAGACCATAGAG | 31 |
| | NotI F | tgTTTCTCGgcggccgcTCAGAACTTGCGCCGATAAA | 32 |
| | BamHI R | | 33 |
| **glt3** | BglII F | aattgggcccagatctATTGGCGGGTTCGTTACTT | 34 |
| | EcoRI R | tctctgggcggaattcCCTGGAAGAAGGCCGTATTATC | 35 |
| | NotI F | tgTTTCTCGgcggccgcATTGGCGGGTTCGTTACTT | 36 |
| | BamHI R | | 37 |

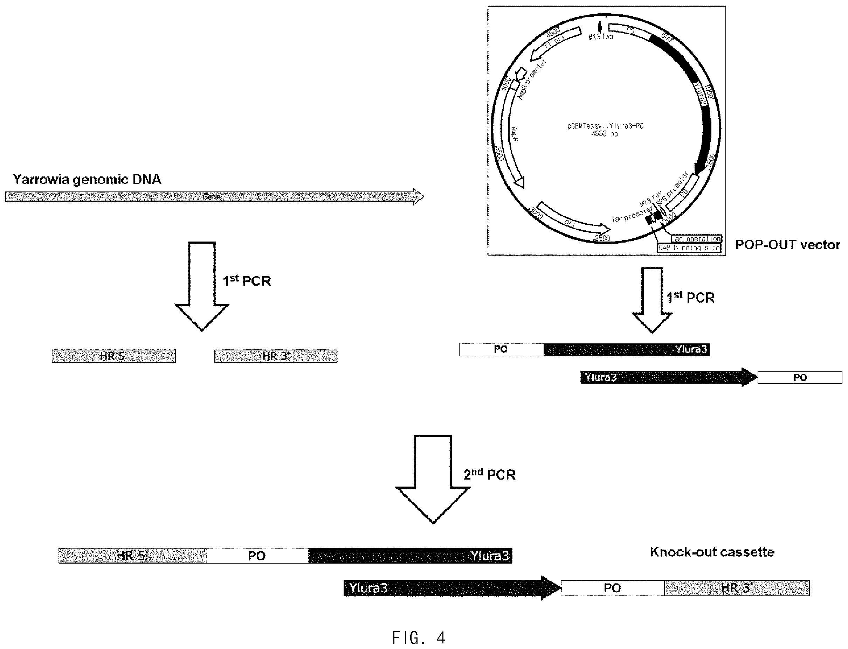

A knock-out cassette was constructed as shown in FIG. 4. First, PCR of a homologous region (HR) to be knocked out from the genomic DNA of *Yarrowia* sp., and PCR of two 5'- and 3'-terminal fragments from a pop-out vector were carried out. Thereafter, each of the 5' HR and 3' HR was subjected to alignment PCR (2^{nd} PCR) with a PO-ura3 region to construct a knock-out cassette. The primers used to amplify the respective homologous regions, and sequences thereof are listed in Table 2.

**[Table 2]**

| Gene Deletions | | | |
|---|---|---|---|
| Names | | **Base Sequences** | **SEQ ID NOs** |
| **ACO1** | F1 | TTCCTCAATGGTGGAGAAGA | 38 |
| | R1 | | 39 |
| | F2 | | 40 |
| | R2 | AAGAAGGGCTTGAGAGTCG | 41 |
| **ACO2** | F1 | CCCAACAACACTGGCAC | 42 |
| | R1 | | 43 |
| | F2 | ATCGCTACCTCATATCCGCACCTCC gacaagacccgacaggc | 44 |
| | R2 | AGACCAGAGTCCTCTTCG | 45 |
| **ACO3** | F1 | Accttcacagagccaccca | 46 |
| | R1 | ATGGCTCTCTGGGCGgtgttgggggtgttgatgatg | 47 |
| | F2 | TTGTTGTGTTTCTCGcaaggttctcatcgaggcctg | 48 |
| | R2 | Aggaaaggtcgaagagtgctct | 49 |
| **ACO4** | F1 | Actgcgagagcgatctg | 50 |
| | R1 | | 51 |
| | F2 | | 52 |
| | R2 | AGAGCAGAGTCCTCCTCAA | 53 |
| **ACO5** | F1 | AACTTCCTCACAGGCAGCGAGC | 54 |
| | R1 | | 55 |
| | F2 | ttgttgtgtttctcg ccccgtcaaggacgctgag | 56 |
| | R2 | ACAGTAAGGTGGGGCTTGACTC | 57 |
| **ACO6** | F1 | AGTCCCTCAACACGTTTACCG | 58 |
| | R1 | | 59 |
| | F2 | | 60 |
| | R2 | AGGAAGGGTCTAATGACAGA | 61 |
| **FALD H1** | F1 | AATCACTCCTCCTACGC | 62 |
| | R1 | | 63 |
| | F2 | | 64 |
| | R2 | ACCGACATAATCTGAGCAAT | 65 |
| **FALD H2** | F1 | Accactaggtgagatcgag | 66 |
| | R1 | | 67 |
| | F2 | | 68 |
| | R2 | GATCACCCAGAACCATAGC | 69 |
| **FALD H3** | F1 | GTGACCCCCACCACGTCAC | 70 |
| | R1 | | 71 |
| | F2 | | 72 |
| | R2 | CAGGGCTGGGGACCACC | 73 |
| **FALD H4** | F1 | TACCGACTGGACCAGATTC | 74 |
| | R1 | | 75 |
| | F2 | | 76 |
| | R2 | CAAATCTTTCGGAAGATTCGG | 77 |
| **FAO1** | F1 | atcattgtcggtggaggaac | 78 |
| | R1 | | 79 |
| | F2 | attctggtactgccgatcgagaaga ccgtcatcggtgagattctt | 80 |
| | R2 | attcgaggtcggagatcctt | 81 |
| **ADH1** | F1 | cccagaaggctgtcattttc | 82 |
| | R1 | ACGCCTTTCTGGTCGAGGTAGCGTTtcgcagttcttggggatatg | 83 |
| | F2 | attctggtactgccgatcgagaaga gccgacaaggagaagatgtg | 84 |
| | R2 | caatcttgccctcctccat | 85 |
| **ADH2** | F1 | ccagaagggtgtcatcttcg | 86 |
| | R1 | ACGCCTTTCTGGTCGAGGTAGCGTTatcgcagttcttgggaatgt | 87 |
| | F2 | attctggtactgccgatcgagaaga ccgacaaggagaagatgtgc | 88 |
| | R2 | caatcttgccctcctccata | 89 |
| **ADH3** | F1 | agaaagccgtcatcttcgag | 90 |
| | R1 | ttgcacaagtaacgaacccgccaat tcacagttcttggggatgtg | 91 |
| | F2 | ggagataatacggccttcttccagg gctgacaaggagaagatgtgc | 92 |
| | R2 | acttggagcagtccagaacg | 93 |
| **ADH4** | F1 | gtcaaaacgtcgacgaacct | 94 |
| | R1 | AGGTATTTATCGGCGCAAGTTCTGA ggcttgaggtcaatgtcgat | 95 |
| | F2 | ctcctctatggtctagctggcaaag gacatggaggcccactctaa | 96 |
| | R2 | agtactcccaagcgtcctca | 97 |
| **ADH5** | F1 | gagagccgctttcaccac | 98 |
| | R1 | | 99 |
| | F2 | ctcctctatggtctagctggcaaag ttccaggacgtgatcaagga | 100 |
| | R2 | taaggatgatcttgccggtag | 101 |
| **ADH6** | F1 | gacccagaaagccattgtgt | 102 |
| | R1 | | 103 |
| | F2 | ctcctctatggtctagctggcaaag caccgaggagaaggagaaga | 104 |
| | R2 | tccctcctccatcaaggtaa | 105 |
| **ADH7** | F1 | gacgttcccaagacacaaaag | 106 |
| | R1 | | 107 |
| | F2 | ctcctctatggtctagctggcaaag acccacaccaaggagctg | 108 |
| | R2 | caacgacacgaccaacaatc | 109 |
| **ADH8** | F1 | atcgcgccaacttgtttaat | 110 |
| | R1 | AGGTATTTATCGGCGCAAGTTCTGA caccttctctcgtgggatgt | 111 |
| | F2 | ctcctctatggtctagctggcaaag tgtgttgagtctggcaaagc | 112 |
| | R2 | tcaagtccatggcatcaaac | 113 |
| **FADH** | F1 | ccgaaggaaagaccatcact | 114 |
| | R1 | ttgcacaagtaacgaacccgccaat agaaggaagagcagcccata | 115 |
| | F2 | ggagataatacggccttcttccagg gcttgggcttacaagtttgg | 116 |
| | R2 | tcggtgaaggcagagttgat | 117 |

The primers used to PCR-amplify the pop-out region and ura3 as two fragments are listed in Table 3.

**[Table 3]**

| Pop-out Cassettes | | | |
|---|---|---|---|
| Names | | **Base Sequences** | **SEQ ID NOs** |
| **HISG1** | F | cagaccggttcagacaggat | 118 |
| | R | ggaggtgcggatatgaggta | 119 |
| **HISG2** | F | aacgctacctcgaccagaaa | 120 |
| | R | tcttctcgatcggcagtacc | 121 |
| **glt2** | F | TCAGAACTTGCGCCGATAAA | 122 |
| | R | CTTTGCCAGCTAGACCATAGAG | 123 |
| **glt3** | F | ATTGGCGGGTTCGTTACTT | 124 |
| | R | CCTGGAAGAAGGCCGTATTATC | 125 |
| **Bipartite** | Ulura3 cs 2B | atgccctcctacgaagctcgagc | 126 |
| | Ylura3F | ctcccaacgagaagctggcc | 127 |

The gene sequences used to modify the recombinant microorganism strain according to the present invention are listed in the sequence listing, and summarized in Table 4.

**[Table 4]**

| **Genes** | **SEQ ID NOs** | **Genes** | **SEQ ID NOs** |
|---|---|---|---|
| ADH1 | 1 | FALDH2 | 12 |
| ADH2 | 2 | FALDH3 | 13 |
| ADH3 | 3 | FALDH4 | 14 |
| ADH4 | 4 | ACO1 | 15 |
| ADH5 | 5 | ACO2 | 16 |
| ADH6 | 6 | ACO3 | 17 |
| ADH7 | 7 | ACO4 | 18 |
| ADH8 | 8 | ACO5 | 19 |
| FADH | 9 | ACO6 | 20 |
| FAO1 | 10 | Ura3 | 21 |
| FALDH1 | 11 | | |

### Example 2: Construction of knock-out strain

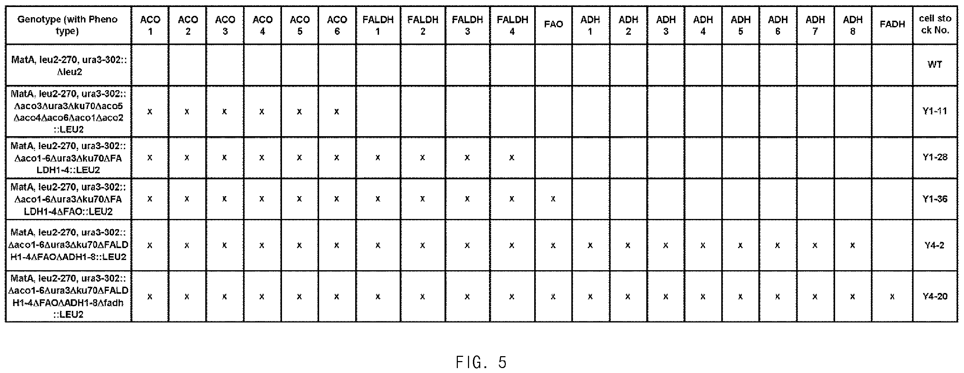

The knock-out cassette constructed in Example 1 was used to prepare a total of six knock-out strains from which some or all of fatty alcohol dehydrogenase, fatty alcohol oxidase and fatty aldehyde dehydrogenase genes and β-oxidative metabolism pathway-related genes present in a wild-type *Yarrowia* strain were deleted (FIG. 5). Specifically, a strain to be knocked out was plated on an YPD plate, and cultured at 30 °C for 16 to 24 hours. The cultured cells were scraped with a loop, put into 100 µL of a one-step buffer (45% PEG4000, 100 mM DTT, 0.1 L LiAc, 25 µg of single-strand carrier DNA), and vortexed. Thereafter, the knock-out cassette (1 ng or more) was added thereto, and the resulting mixture was vortexed again, and cultured at 39 °C for an hour. The cultured sample was loaded onto a selective medium (6.7 g/L of YNB without amino acids, and 20 g/L of glucose), and then cultured at 30 °C for 48 hours to screen a strain into which the constructed cassette was inserted. To check whether the cassettes were correctly inserted onto the genome of the screened strain, PCR was then performed using the primers included in the gene deletions listed in Table 2.

To insert another cassette, a pop-out process was performed on the strain into which the cassette was inserted. The strain screened from a selective medium was inoculated in 2 mL of an YPD medium, and cultured at 30 °C for 16 hours, and 200 µL of the culture broth was spread on a 5' FOA medium (6.7 g/L of YNB without amino acids, 20 g/L of glucose, 0.8 g/L of 5' FOA, 0.1 g/L of uracil, and 0.1 g/L of uridine), and then cultured at 30 °C for 48 hours. The strains grown on the 5' FOA medium were picked, and plated on an YPD plate and a UD plate. Then, the strains grown on the YPD plate were screened, and a PCR process was again performed using the primers listed in Table 2 to check whether the ura3 gene was deleted from the strains. A knock-out process was performed on other genes of the Ura3-deleted strains.

The aforementioned processes were repeatedly performed to prepare the recombinant microorganism strain of the present invention (Chen DC, Beckerich JM, Gaillardin C (1997) Appl Microbiol Biotechnol 48: 232-235).

### Example 3: Culturing of knock-out strain

A day earlier, the strain to be cultured and tested was inoculated in 2 mL of an YPD medium (Bacto Laboratories, 10 g/L of Yeast extract, 20 g/L of peptone, and 20 g/L of glucose), and grown at 30 °C and 200 rpm for a day. 2 mL of a growth medium (pH 6.0) having the compositions listed in Table 5 was put into a 24-well plate, and a pre-cultured culture broth was inoculated at 1%. Thereafter, the strains were cultured at 30 °C and 450 rpm for a day in a plate stirrer. The strains cultured for a day were inoculated at a volume of 900 µL in a new plate containing 900 µL of a conversion medium (pH 7.6) listed in Table 6, and 200 µL of a substrate was added thereto at the same time. The resulting mixture was cultured at 30 °C and 450 rpm for a day. In this case, 10 g/L of dodecane dissolved in DMSO was used as the substrate.

**[Table 5]**

| **Growth Medium (pH 6.0)** | | |
|---|---|---|
| Components | | Concentration (g/L) |
| Glucose | | 50 |
| YNB w/o amino acids | | 6.7 |
| Yeast extract | | 10 |
| (NH₄)₂SO₄ | | 5 |
| Uracil | | 0.05 |
| 0.1 M phosphate buffer | | |

| Preparation of 0.1 M potassium phosphate buffer at 25 °C | | |
|---|---|---|
| pH | Volume (mL) of 1 M K₂HPO₄ | Volume (mL) of 1 M KH₂PO₄ |
| 6.0 | 13.2 | 86.8 |

**[Table 6]**

| **Conversion Medium (pH 7.6)** | | |
|---|---|---|
| Components | | Concentration (g/L) |
| Glucose | | 30 |
| YNB w/o amino acids | | 6.7 |
| Yeast extract | | 3 |
| (NH₄)₂SO₄ | | 15 |
| Uracil | | 0.05 |
| L-alanine | | 10 |
| 0.1 M phosphate buffer | | |

| Preparation of 0.1 M potassium phosphate buffer at 25 °C | | |
|---|---|---|
| pH | Volume (mL) of 1 M K₂HPO₄ | Volume (mL) of 1 M KH₂PO₄ |
| 7.6 | 86.6 | 13.4 |

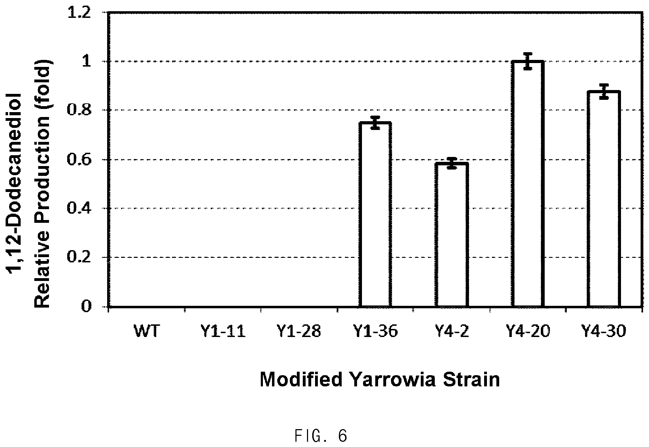

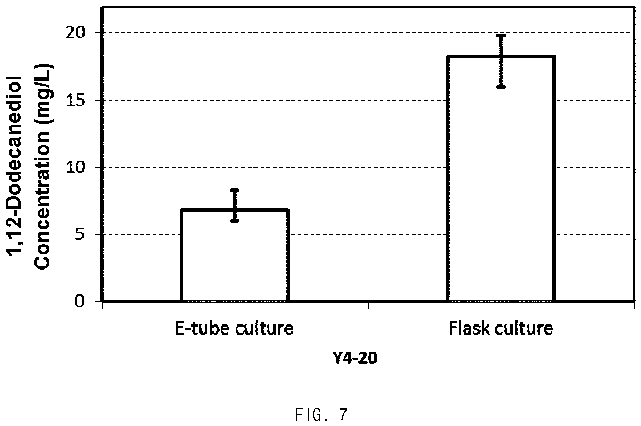

As a result, it was revealed that the Y1-28 and Y1-36 strains in which only the β-oxidative metabolism pathway-related genes and the fatty aldehyde dehydrogenase gene were knocked out did not produce 1,12-dodecanediol from dodecane serving as the substrate, but all the Y1-36 strain in which the fatty alcohol oxidase gene was further knocked out, and the Y4-2, Y4-20 and Y4-30 strains in which the fatty alcohol oxidase gene and the fatty alcohol dehydrogenase gene were further knocked out exhibited an excellent ability to synthesize 1,12-dodecanediol (FIG. 6). Also, it was revealed that the Y4-20 strain exhibited an ability to synthesize approximately 18 mg/L of 1,12-dodecanediol when cultured in the flask (FIG. 7). In the following experiment, a sample analysis test was performed using the Y4-20 strain.

### Example 4: Sample Analysis

1 mL of 1 N sodium hydroxide and 10 mL of chloroform were added to 10 mL of a culture broth of the Y4-20 strain which had been proven to have the most excellent ability to synthesize 1,12-dodecanediol in Example 3. Thereafter, the resulting mixture was thoroughly vortexed, extracted, and then centrifuged at 10,000 rpm for 10 minutes. Then, only a chloroform layer was separated, concentrated 10-fold, and then subjected to a GC/MS assay under the following analytical conditions.

### Analytical Conditions

① Equipment: Agilent 5975 MSD
② Column: HP-5MS
③ Temperature: Oven (150 °C to 230 °C)
④ Carrier gas: He
⑤ Flow rate: 1 mL/min.

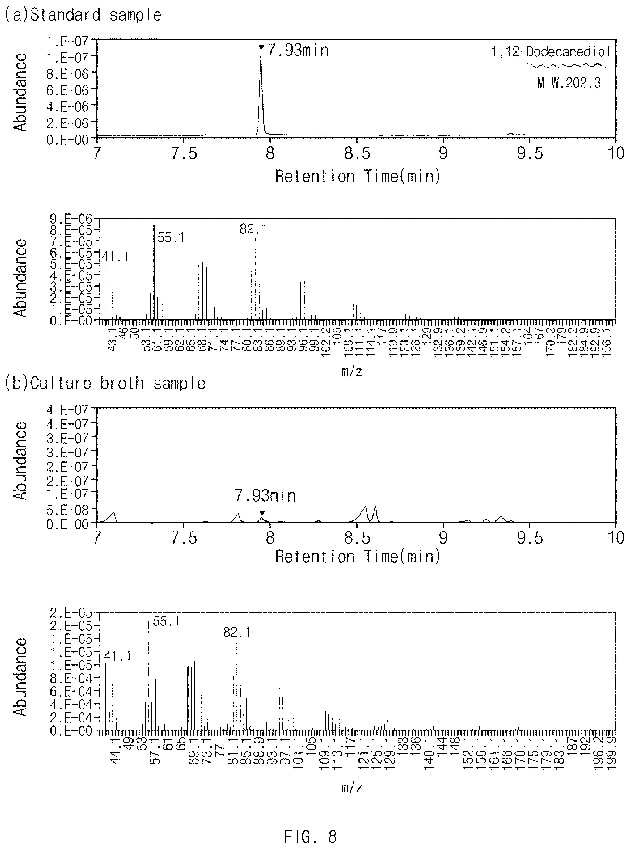

As a result, it was confirmed that the recombinant Y4-20 strain of the present invention was able to synthesize 1,12-dodecanediol from dodecane serving as a substrate (FIG. 8).
## Claims

1. A recombinant microorganism from which the fatty alcohol dehydrogenase, the fatty alcohol oxidase and the fatty aldehyde dehydrogenase genes in the ω-oxidative metabolism pathway, and the β-oxidative metabolism pathway-related genes are deleted.

2. The recombinant microorganism of claim 1, wherein the fatty alcohol dehydrogenase gene, the fatty alcohol oxidase gene, the fatty aldehyde dehydrogenase gene, and the β-oxidative metabolism pathway-related genes are deleted from all homologous genes present in the microorganism.

3. The recombinant microorganism of claim 1, wherein the fatty alcohol dehydrogenase gene, the fatty alcohol oxidase gene, the fatty aldehyde dehydrogenase gene, and the β-oxidative metabolism pathway-related genes are deleted from some of the homologous genes present in the corresponding microorganism.

4. The recombinant microorganism of claim 1, wherein the fatty alcohol dehydrogenase gene is selected from the group consisting of ADH1, ADH2, ADH3, ADH4, ADH5, ADH6, ADH7, ADH8, and FADH genes, and preferably each of the ADH1, ADH2, ADH3, ADH4, ADH5, ADH6, ADH7, ADH8 and FADH genes comprises base sequences set forth in SEQ ID NOs: 1 to 9, respectively..

5. The recombinant microorganism of claim 1, wherein the fatty alcohol oxidase gene is an FAO gene, and preferably the FAO gene comprises a base sequence set forth in SEQ ID NO: 10.

6. The recombinant microorganism of claim 1, wherein the fatty aldehyde dehydrogenase gene is selected from the group consisting of FALDH1, FALDH2, FALDH3, and FALDH4 genes, and preferably each of the FALDH1, FALDH2, FALDH3, and FALDH4 genes comprises base sequences set forth in SEQ ID NOs: 11 to 14, respectively.

7. The recombinant microorganism of claim 1, wherein the β-oxidative metabolism pathway-related gene is an acyl-CoA oxidase gene.

8. The recombinant microorganism of claim 7, wherein the acyl-CoA oxidase gene is selected from the group consisting of ACO1, ACO2, ACO3, ACO4, ACO5, and ACO6 genes, and preferably each of the ACO1, ACO2, ACO3, ACO4, ACO5, and ACO6 genes comprises base sequences set forth in SEQ ID NOs: 15 to 20, respectively.

9. The recombinant microorganism of claim 1, wherein the microorganism is a yeast or *Escherichia coli.*

10. The recombinant microorganism of claim 9, wherein the yeast is selected from the group of the yeast consisting of *Yarrowia* sp., *Saccharomyces* sp., *Pichia* sp., and *Candida* sp.

11. The recombinant microorganism of claim 10, wherein the yeast of *Yarrowia* sp. is *Yarrowia lipolytica.*

12. A method for producing a medium chain diol, comprising:
(1) preparing the recombinant microorganism according to any one of claims 1 to 11; and
(2) treating the recombinant microorganism with a substrate to culture the recombinant microorganism.

13. The method of claim 12, wherein the substrate is selected from the group consisting of a fatty acid-derived alcohol and alkane.

14. The method of claim 13, wherein the fatty acid-derived alcohol or alkane has 5 to 30 carbon atoms.

15. The method of claim 12, wherein the medium chain diol is a diol compound having 5 to 30 carbon atoms.

## Patentansprüche

1. Ein rekombinanter Mikroorganismus aus dem die Fettalkohol- Dehydrogenase-, die Fettalkohol- Oxidase- und die Fettaldehyd- Dehydrogenase- Gene im ω-oxidativen Stoffwechselweg, und die mit dem β-oxidativen Stoffwechselweg zusammenhängenden Gene entfernt werden.

2. Der rekombinante Mikroorganismus nach Anspruch 1, wobei das Fettalkohol-Dehydrogenasegen, das Fettalkohol- Oxidasegen, das Fettaldehyd-Dehydrogenasegen und die mit dem β-oxidativen Stoffwechselweg zusammenhängenden Gene aus allen im Mikroorganismus vorhandenen homologen Genen entfernt werden.

3. Der rekombinante Mikroorganismus nach Anspruch 1, wobei das Fettalkohol-Dehydrogenasegen, das Fettalkohol- Oxidasegen, das Fettaldehyd-Dehydrogenasegen und die mit dem β-oxidativen Stoffwechselweg zusammenhängenden Gene aus einigen im entsprechenden Mikroorganismus vorhandenen homologen Genen entfernt werden.

4. Der rekombinante Mikroorganismus nach Anspruch 1, wobei das Fettalkohol-Dehydrogenasegen ausgewählt wird aus der Gruppe bestehend aus ADH1-, ADH2-, ADH3-, ADH4-, ADH5-, ADH6-, ADH7-, ADH8- und FADH- Genen und vorzugsweise jedes der ADH1-, ADH2-, ADH3-, ADH4-, ADH5-, ADH6-, ADH7-, ADH8- und FADH- Gene eine Basensequenz umfasst, wie jeweils festgelegt in SEQ ID NO: 1 bis 9.

5. Der rekombinante Mikroorganismus nach Anspruch 1, wobei das Fettalkohol-Oxidase Gen ein FAO- Gen ist und das FAO- Gen eine Basensequenz wie festgelegt in SEQ ID NO: 10 umfasst.

6. Der rekombinante Mikroorganismus nach Anspruch 1, wobei das Fettalkohol-Dehydrogenasegen ausgewählt wird aus der Gruppe bestehend aus FALDH1-, FALDH2-, FALDH3- und FALDH4- Genen und vorzugsweise jedes der FALDH1-, FALDH2-, FALDH3- und FALDH4- Gene eine Basensequenz umfasst, wie jeweils festgelegt in SEQ ID NO: 11 bis 14.

7. Der rekombinante Mikroorganismus nach Anspruch 1, wobei das mit dem β-oxidativen Stoffwechselweg zusammenhängende Gen ein Acyl-CoA- Oxidasegen ist.

8. Der rekombinante Mikroorganismus nach Anspruch 7, wobei das Acyl-CoA-Oxidasegen ausgewählt wird aus der Gruppe bestehend aus ACO1-, ACO2-, ACO3-, ACO4-, ACO5- und ACO6- Genen und vorzugsweise jedes der ACO1-, ACO2-, ACO3-, ACO4-, ACO5- und ACO6- Gene eine Basensequenz umfasst, wie jeweils festgelegt in SEQ ID NO: 15 bis 20.

9. Der rekombinante Mikroorganismus nach Anspruch 1, wobei es sich bei dem Mikroorganismus um eine Hefe oder *Escherichia coli* handelt.

10. Der rekombinante Mikroorganismus nach Anspruch 9, wobei die Hefe ausgewählt wird aus der Gruppe bestehend aus *Yarrowia sp., Saccharomyces sp., Pichia sp.* und *Candida sp.*

11. Der rekombinante Mikroorganismus nach Anspruch 10, wobei es sich bei der *Yarrowia sp.* Hefe um *Yarrowia lipolytica* handelt.

12. Ein Verfahren zur Herstellung eines mittelkettigen Diols, das umfasst:
Herstellung des rekombinanten Mikroorganismus nach irgendeinem der Ansprüche 1 bis 11, und
Behandlung des rekombinanten Mikroorganismus mit einem Substrat zur Kultivierung des rekombinanten Mikroorganismus.

13. Verfahren nach Anspruch 12, wobei das Substrat ausgewählt wird aus der Gruppe bestehend aus Fettsäure-Derivat-Alkohol und Alkan.

14. Verfahren nach Anspruch 13, wobei der Fettsäure-Derivat-Alkohol oder das Alkan 5 bis 30 Kohlenstoffatome enthält.

15. Verfahren nach Anspruch 12, wobei es sich bei dem mittelkettigen Diol um eine Diol- Verbindung mit 5 bis 30 Kohlenstoffatomen handelt.

## Revendications

1. Micro-organisme recombinant dans lequel les gènes de la déshydrogénase des alcools gras, de l'oxydase des alcools gras et de la déshydrogénase des aldéhydes gras de la voie métabolique ω-oxydative, et les gènes relatifs à la voie métabolique β-oxydative sont délétés.

2. Micro-organisme recombinant selon la revendication 1, dans lequel le gène de la déshydrogénase des alcools gras, le gène de l'oxydase des alcools gras et le gène de la déshydrogénase des aldéhydes gras, et les gènes relatifs à la voie métabolique β-oxydative sont délétés de tous les gènes homologues présents dans le micro-organisme.

3. Micro-organisme recombinant selon la revendication 1, dans lequel le gène de la déshydrogénase des alcools gras, le gène de l'oxydase des alcools gras et le gène de la déshydrogénase des aldéhydes gras, et les gènes relatifs à la voie métabolique β-oxydative sont délétés de certains des gènes homologues présents dans le micro-organisme correspondant.

4. Micro-organisme recombinant selon la revendication 1, dans lequel le gène de la déshydrogénase des alcools gras est sélectionné parmi le groupe constitué des gènes ADH1, ADH2, ADH3, ADH4, ADH5, ADH6, ADH7, ADH8 et FADH, et de préférence chacun des gènes ADH1, ADH2, ADH3, ADH4, ADH5, ADH6, ADH7, ADH8 et FADH comprend des séquences en bases décrites dans SEQ ID NO: 1 à 9, respectivement.

5. Micro-organisme recombinant selon la revendication 1, dans lequel le gène de l'oxydase des alcools gras est un gène FAO, et de préférence le gène FAO comprend une séquence en bases décrite dans SEQ ID NO: 10.

6. Micro-organisme recombinant selon la revendication 1, dans lequel le gène de la déshydrogénase des aldéhydes gras est sélectionné parmi le groupe constitué des gènes FALDH1, FALDH2, FALDH3 et FALDH4, et de préférence chacun des gènes FALDH1, FALDH2, FALDH3 et FALDH4 comprend des séquences en bases décrites dans SEQ ID NO: 11 à 14, respectivement.

7. Micro-organisme recombinant selon la revendication 1, dans lequel le gène relatif à la voie métabolique β-oxydative est un gène d'acyl-CoA oxydase.

8. Micro-organisme recombinant selon la revendication 7, dans lequel le gène d'acyl-CoA oxydase est sélectionné parmi le groupe constitué des gènes ACO1, ACO2, ACO3, ACO4, ACO5 et ACO6, et de préférence chacun des gènes ACO1, ACO2, ACO3, ACO4, ACO5 et ACO6 comprend des séquences en bases décrites dans SEQ ID NO: 15 à 20, respectivement.

9. Micro-organisme recombinant selon la revendication 1, dans lequel le micro-organisme est une levure ou *Escherichia coli.*

10. Micro-organisme recombinant selon la revendication 9, dans lequel la levure est sélectionnée parmi le groupe de levures constitué de *Yarrowia* sp., *Saccharomyces* sp., *Pichia* sp. et *Candida* sp.

11. Micro-organisme recombinant selon la revendication 10, dans lequel la levure *Yarrowia* sp. est *Yarrowia lipolytica.*

12. Méthode de production d'un diol à chaîne moyenne, comprenant:
(1) la préparation du micro-organisme recombinant selon l'une quelconque des revendications 1 à 11; et
(2) le traitement du micro-organisme recombinant avec un substrat pour cultiver le micro-organisme recombinant.

13. Méthode selon la revendication 12, dans lequel le substrat est sélectionné parmi le groupe constitué d'un alcool dérivé d'acide gras et un alcane.

14. Méthode selon la revendication 13, dans lequel l'alcool dérivé d'acide gras ou l'alcane a de 5 à 30 atomes de carbone.

15. Méthode selon la revendication 12, dans lequel le diol à chaîne moyenne est un composé diol ayant 5 à 30 atomes de carbone.


<110> Korea Research Institute of Bioscience and Biotechnology
<120> METHOD FOR PRODUCTION OF MEDIUM CHAIN DIOL
<130> 2016-OPA-8749PCT
<150> KR 10-2015-0149251
   <151> 2015-10-27
<160> 127
<170> KopatentIn 2.0
<210> 1
   <211> 1050
   <212> DNA
   <213> Yarrowia lipolytica
<220>
   <221> gene
   <222> (1)..(1050)
   <223> ADH1 gene
<400> 1
   atgaccacca tccccaagac ccagaaggct gtcattttcg agacctccgg cggtcctctc      60
   atgtacaagg acgtgcctgt gcctgttcct gccgacgacg agattctcgt caacgtcaag     120
   ttttccggag tgtgccacac tgacctccat gcctggaagg gcgactggcc tcttgacacc     180
   aaacttcctc tcattggcgg ccacgagggc gccggagttg tcgtggccaa gggcaagaac     240
   gtgaccacct tcgaaatcgg cgactacgcc ggtatcaagt ggattaacaa ggcctgttac     300
   acctgtgagt tctgccaggt gtctgccgag cccaactgtc ctaaagctac catgtcggga     360
   tacacccacg acggctcgtt ccagcagtac gccactgcca acgctgtcca ggcagcccat     420
   atccccaaga actgcgacct cgcccagatt gctcccattc tctgcgccgg catcaccgtc     480
   tacaaggctc ttaagaccgc tggcctcaag gctggtgagt gggccgccgt taccggagct     540
   ggaggaggcc tcggttctct ggccgtccag tacgctaagg ccatgggcta ccgagtgctg     600
   gccattgaca ccggcgccga caaggagaag atgtgcaagg agctcggcgc cgaggtcttc     660
   atcgactttg ctaagtccaa ggatctggtc aaggacgtcc aggatgccac caagggcgga     720
   ccccacgccg ttatcaacgt gtctgtctcc gagtttgccg tcaaccagtc cgtcgagtac     780
   gtgcgaactc tgggcaccgt ggttctggtc ggcctgcccg ctggagctgt ctgcaagtcg     840
   cccatcttcc agcaagtggc tcgatccatt cagatcaagg gttcctatgt tggcaaccga     900
   gccgactcgc aggaggccat tgaattcttc gcccggggcc ttgtcaagtc tcccattatt     960
   attgtgggtc tttcccagct ggagtccgtc tacaagctga tggaggaggg caagattgcc    1020
   ggcagatacg ttctggacac ttacaagtaa                                     1050
<210> 2
   <211> 1056
   <212> DNA
   <213> Yarrowia lipolytica
<220>
   <221> gene
   <222> (1)..(1056)
   <223> ADH2 gene
<400> 2
   atgtctgctc ccgtcatccc caagacccag aagggtgtca tcttcgagac ctccggcggt 60
   cctctcatgt acaaggacat ccccgtgcct gtgcctgccg acgacgagat tctggtcaac 120
   gtcaagttct ccggagtctg ccacacggat ctgcacgcct ggaagggcga ctggcctctg 180
   gacaccaagc ttcctctggt cggaggccac gagggtgccg gagtggttgt tgccaagggt 240
   aagaacgttg acacgtttga gattggcgac tatgccggca tcaagtggat caacaaggcc 300
   tgctacacct gcgagttctg ccaggtggcc gccgagccca actgtcccaa cgctaccatg 360
   tctggataca cccacgacgg ctctttccag cagtacgcca ccgccaacgc cgtgcaggcc 420
   gcgcacattc ccaagaactg cgatctcgcc gagattgccc ccattctgtg cgccggaatc 480
   accgtctaca aggctctcaa gactgccgcc atcctcgctg gccagtgggt tgccgttact 540
   ggtgctggag gaggactcgg aacacttgct gtccagtacg ccaaggccat gggctaccga 600
   gtgctggcca ttgacactgg cgccgacaag gagaagatgt gcaaggacct tggtgccgag 660
   gttttcatcg actttgccaa gaccaaggac ctcgtcaagg acgtccagga ggccaccaag 720
   ggcggacccc acgccgtcat caatgtgtct gtctccgagt ttgcagtcaa ccagtccatt 780
   gagtacgtgc gaaccctggg aaccgttgtt ttggtcggtc tgcccgccgg cgccgtctgc 840
   aagtctccca tcttccagca ggtggctcga tctatccaga tcaagggctc ttacgttgga 900
   aaccgagccg actcccagga ggccattgag ttcttctccc gaggtctcgt caagtcgccc 960
   atcatcatca tcggtctgtc cgagctggaa aaggtctaca agcttatgga ggagggcaag 1020
   attgccggcc gatacgttct ggacacctcc aagtaa 1056
<210> 3
   <211> 1050
   <212> DNA
   <213> Yarrowia lipolytica
<220>
   <221> gene
   <222> (1)..(1050)
   <223> ADH3 gene
<400> 3
   atgaccacca tccccaagac ccagaaagcc gtcatcttcg agacctccgg cggccccctc 60
   atgtacaagg acgtgcctgt gcccgtgcct gccgacgacg agattctggt caacgtcaag 120
   tactccggcg tgtgccacac ggacctgcac gcctggaagg gcgactggcc cctggacacc      180
   aagctccccc tgattggcgg ccacgagggc gccggcgtgg ttgttgccaa gggcaagaac      240
   gtgaccacct ttgagattgg cgactacgcc ggtatcaagt ggatcaacaa ggcctgctac      300
   acctgcgagt tctgccaggt ggcggccgag cccaactgcc ccaaggccac catgtccgga      360
   tacacccacg acggctcttt ccagcagtac gccaccgcca acgctgtcca ggccgcccac      420
   atccccaaga actgtgacct cgcccaggtt gcccccattc tctgcgccgg tatcaccgtc      480
   tacaaggctc tcaagaccgc tggcctcaag gctggtgagt gggccgccgt gaccggagct      540
   ggaggaggcc tcggctctct ggccgtccag tacgccaagg ccatgggcta ccgagtgctg      600
   gccattgaca ctggcgctga caaggagaag atgtgcaagg agctcggcgc cgaggtcttc      660
   atcgactttg ccaagtccaa ggatctggtc aaggacgtcc aggaggccac caagggcgga      720
   ccccacgccg tcatcaacgt gtctgtctcc gagtttgccg tcaaccagtc tgttgagtac      780
   gtgcgaaccc tgggaaccgt tgttctggtc ggtctgcccg ccggtgccgt ctgcaagtcg      840
   cccatcttcc agcaggttgc tcgatctatt cagatcaagg gctcttacgt cggaaaccga      900
   gccgactccc aggaggccat tgagttcttt gcccgaggac tggtcaagtc ccccatcatt      960
   attgttggtc tctccgagct cgaatccgtc tacaagctca tggaggaggg caagattgcc     1020
   ggtcgatacg ttctggactg ctccaagtaa                                      1050
<210> 4
   <211> 1479
   <212> DNA
   <213> Yarrowia lipolytica
<220>
   <221> gene
   <222> (1)..(1479)
   <223> ADH4 gene
<400> 4
   atggtcaaaa cgtcgacgaa ccttgcacga aacgtgctgc gaaccatcca ggccaaccct      60
   cctcccggcc tcgaggtaaa tggcatcacc tcacacaact tcggagccta ccagcgggct     120
   ttcaccccca tggtgatgca gggagccgga cacagaaact acgccagtga tcacaaggag    180
   acggagtacg cgttccagat ggccgcttcc aacatccgat acggccccgg cgtgacggca     240
   gaggtgggct acgacttcaa gaacatgcga atcgaccggg tggctgtctt caccgacaag     300
   aacctgctca atacccccgc cgtcaaaacc gcactgcagt cgctcgacaa gtgtggaatc    360
   aaatacgacc tttattcgga cgtgtgcgtt gagcccaaag agccctctgt gctcgacgcc     420
   attgcctggg gacgagctaa gcagcccaag gcctaccttg ccattggagg aggctctgtc     480
   atggacactg ccaagatggc caacctgtac cagtgcttcc ccgacgctga gctgcttgat     540
   ttcgtcaacg cccccattgg aaaggctcag cccatcgaca ttgacctcaa gcctctgatt     600
   gccgtcccca ccactgccgg caccggatcc gagaccactg gaactgccat cttcgatctc     660
   gtttcccgaa aggccaagac cggtattgct aaccgtgctc tgaagcccct gcttggaatt     720
   gtcgatcctc tcaactctgc caccatgccc gagcaggtta aggccgcttc cggcctcgat     780
   gtcctatgtc actctcttga gtcctacact gccatcccct accagcagcg aaccccccgt    840
   cccagcaacc ccaacatgcg acctgcctac caaggatcca accccattgc tgatattttc     900
   tctctcgagg gtctgcgtct ggccattgag taccttcctc gatcttgtgc cgaccccgaa     960
   gacatggagg cccactctaa catgcttctt ggatctactc ttgccggtgt gggttttgga    1020
   aacgccggtg ttcacatctg ccacggtctc tcttacccca tctctggaat gaacacctcc    1080
   tactaccacc ccgactacca cactgaccat cctctggttc cccacggtat ctccgttgca    1140
   gttactgctc cttctgtctt caagtttact gctcccagcg accccgagcg acatctcaag    1200
   gccgcctctc tgtttggcgt tgatgtttcc aacgtcaagc gagagtctgc cggagaggtg    1260
   cttgctgagg ccatccaaga gttcatgttc actaagctca agcaccaacc ccggggtgtc    1320
   tctgctcttg gttacaagcg aagcgacatt tctgctctcg ttgacggtgc cgttcctcaa    1380
   cgacgagttc ttgatctcgc ccccggtatc cacggtgttg aggaggccga ggtccgagag     1440
   gcccttacct ctattcttga ggacgcttgg gagtactaa                           1479
<210> 5
   <211> 1041
   <212> DNA
   <213> Yarrowia lipolytica
<220>
   <221> gene
   <222> (1)..(1041)
   <223> ADH5 gene
<400> 5
   atgagagccg ctttcaccac cgcgtacggc ggtcccgaca agatcgagta ctccgattct      60
   ctgcccaagg tgaagcttgg aggtgacgac cacgtgctca tccgagttgc cgacgcctcc     120
   atcaacccca ttgatgggct gcgaaaccga ggaatgctgc gtattctcat gtgtgacgac     180
   catccccacg tctttggata cgacgtcgga ggcttcgtcg aagaggtcgg ctccaagtgc     240
   accaacctca aggttggcga ccgagtctac ggccgaattg gcgagtctca gagcggcacc     300
   ctcgccggct acgtttctgc ccaggagagc gtcattgccg tggctcctac taacctgcct     360
   ctgagcgaga ctgctggtgt gcctttggtt ggtctcactg cctaccaggc tctgagagcg     420
   ggcgatgtgc agcctggaca gcgagtcttc atctccaagg gcgctggagg agttggaacc     480
   atcgctatcc agctcgccaa gcacgtcttt ggagcttatg tcattactac tggttcggac     540
   cacaaggccg agcttctcaa agagcttgga gctgacgagg tcatcaacta ccgaaaggaa     600
   aagttccagg acgtgatcaa ggagccagtc gactttgcct ttgacgtttc cgacgagcct     660
   gccgcacatg caaagatcac caagaagaac ggattcgtgg cggctctgcg aggagctcct     720
   tctcccgcta ctgccaagaa gattctggcc caccctcctg gattcctcat gaacaacgtt     780
   ctgcgagccg ctaactttgc aacctcccga actgcctggt ggtacggagt ccgatacgag     840
   gccatctact gcgttccttc tgctaaggat ctggacactc tgcgaggcta ccttgaaaag     900
   ggtactatca agcccatcgt tgactgcact tatgacctca aggatgccaa gctagcaatg     960
   gagaagcttg agagtggacg agctaccggc aagatcatcc ttagtgtgga tgatactctt    1020
   gataaggagt tcaagcagta a                                               1041
<210> 6
   <211> 1047
   <212> DNA
   <213> Yarrowia lipolytica
<220>
   <221> gene
   <222> (1)..(1047)
   <223> ADH6 gene
<400> 6
   atgacaatcc ccaagaccca gaaagccatt gtgttcgaaa cctctggagg tcccctggag 60
   tacaaggacg tgcctgtgcc cgtgcccggc gaccatcaga ttctcgtcaa cgtcaagtac 120
   tcgggagttt gccactctga cctccacgcc tggatgggag actggcctct acccaccaag 180
   ctccctctta tcggcggcca cgaaggagcc ggcgtcgtgg tggccaaggg aaaaaacgtg 240
   accacgttcg aaattggaga ctacgccgga atcaagtgga tcaaccaggc gtgctacacg 300
   tgcgagttct gccaggaggc ctacgagccc aactgtccca aggcccagat ctcgggatac 360
   acaatggacg gaaccttcca gcagtatgca cttgcagatg ctgtgcaggc agcccacatt 420
   ccccagggca cagacctttc tcaggtggct cctattctgt gtgccggagt gactgtttac 480
   aaggccatca agacctctgg acgaaaggca ggagaatggt tggccgtgac aggtgcagga 540
   ggaggactcg gatcgctggc ggtgcagtac gccaaggcca tgggtttccg agtgctggcc 600
   atcgacacca ccgaggagaa ggagaagatg tgtctggaac tgggagcaga ggtctttgtg 660
   gactttgcca agactgacaa tctggtggca cgagttcagg agattactgg aggtggccct 720
   catggagtca tcaacgtttc tgtgtccgag tttgccatca accagtctct cgagtacgtt 780
   cggtccgttg gaactgttgt tctggtgggt ctgcctgctg gagctgtgtg caagtcgccc 840
   atcttctcgc aggtggcccg ggctattacc atcaagggct ctcctgtggg taaccgagcc 900
   gacacccagg aggctctgtc attctttacc cgaggattgg ttcactcgcc tattcatgtg 960
   gtcggactgt ctgagctgca gaaggtgttt accttgatgg aggagggaaa gattgcgggt 1020
   cggtatgttg tcgataccag caagtaa 1047
<210> 7
   <211> 1065
   <212> DNA
   <213> Yarrowia lipolytica
<220>
   <221> gene
   <222> (1)..(1065)
   <223> ADH7 gene
<400> 7
   atgagcgacg ttcccaagac acaaaaggcc gtcgttttcg aggaagtcaa cggacctttg      60
   atgtacaagg acattcccgt ccccactccc gccaaggacg agctgctcgt caaggtgcag     120
   tattccggtg tctgccactc ggatctgtcc atctggaagg gtgattgggc acagcagctg     180
   cggttcagcc ccaagatgcc gctggtcggc ggtcatgagg gagcaggaga ggttgtgggc     240
   atgggcgatc aggtgaccgg atggcaggtc ggagaccgaa ccggagtcaa gtttatttct     300
   ggctcttgtc tcacttgcga gcactgttct gctggctggg accagcactg cgtagccccc     360
   ggcgtgtcag gtctgctcaa agacggctct ttccagcagt acgcctgcgt gaaggccgcc     420
   accgcacccc gaatccccga ttcttgcgat ctggctggtg ttgcacccgt tctgtgtgca     480
   ggcatcaccg cctacactgc cctcaagaac tctggtctca aggccggtga gtgggtggtg     540
   atcaccggag ctggaggagg actcggatcc tacgccgtcc agtacgccaa gtgcatgggt     600
   ttccgtgtga ttgccattga cactggagac gacaaggaga cccacaccaa ggagctggga     660
   gccgaggtgt ttattgactt tgccaagagt ggtgctggca tgattgctga gattcacaag     720
   ctcaccggag gtggcgccca cgccgtggtc aactttgctg tgcaggacgc ggctgtcgag     780
   gctgccactc tgtacgtgcg aacccgaggc actctggttc tgtgtgctct gccacccaac     840
   ggtaccgtca agagtcacat tctcaaccac gtgggtcgag gactcaccat caagggcagt     900
   tatgtgggta ataagctgga tactcaggaa gccattgact tctatgcacg gggtctcgtc     960
   aagaccaagt accgtctcgg cgagctgagc aagctcgagg agtattacca gcagatgctt    1020
   gatggtaaga ttgttggtcg tgtcgttgtt gataacagca agtag                    1065
<210> 8
   <211> 1275
   <212> DNA
   <213> Yarrowia lipolytica
<220>
   <221> gene
   <222> (1)..(1275)
   <223> ADH8 gene
<400> 8
   atggtattga tcgaatcagg gtatgattat ggctatatac gtcttttcag aatcgcgcca 60
   acttgtttaa ttgaggaatt aaaggccccc ttcttctcgt tccccacctt tgaacgaaca 120
   cactatccaa ccctaactta taagcgaaac cgatctacac aacacatcac ttcctgcgct 180
   tttaccacaa gtcgtcgaca tctcctttac cagcaattac actctctcga catgtctctg 240
   ccactcaccg ataaatcttt caagcgtctg ggcaactctg gcctcaaggt gtcctccatc 300
   attgtgggat gtatgtcgtt tggttctagc aactgggctc cttgggtcat tggagacgaa 360
   gagcggtccc tggagctgct gaaggctgcc tacgaccgag gtttgcgaac gttcgacacc 420
   gcctccacct actccaacgg tctctccgag gtgctactgg gcaagtttct gcgaaagtac 480
   aacatcccac gagagaaggt ggtcattatg accaaggtgt tcttccccgt ggccgaggag 540
   ggactccacg gagagaccat tctcggcggt cgatcggagg aggagatgct ggagttcacc 600
   aaccgaatcg gactgtctcg aaagaacatt attgcctcgg tggacgactg ctgtgaacga 660
   ctcggcactt acattgatct gctgcagatc catcgactcg acgacgagtg tccctacgag 720
   gagatcatga aggccctgca tgactgtgtt gagtctggca aagcccgcta cctaggagcc 780
   tcttccatgc gagccgtgga gtttgtggag ctgcagaacg tggctgagaa gcatggctgg 840
   accaagttta tctctatgca gtctctttac aacctcatca accgggagga cgagcgggaa 900
   ctcaactggt actgcaacaa gactggtgtt ggtctcattc cctggtctcc tctggctcga 960
   ggcattcttg cgcgacccag aagtgctgag gacactgctc ggtccggttc tgatctgcga 1020
   atgatgctgt tcgacaagga ccacgactcc actgccgaga tcatcgaccg ggtcgagaag 1080
   atggccaaga agaagggcgt tgccatggcc acaatcgcca ccgcctgggt ccttcacaag 1140
   ggtgccatgc ccattgttgg tttctcgtcc gagaagcgaa tggatgaggc tctggccgcc 1200
   ctggacgttg agtttgatgc catggacttg aagtacctcg aggatgctta cgagcccgtc 1260
   aagtacaaga tgtaa 1275
<210> 9
   <211> 1146
   <212> DNA
   <213> Yarrowia lipolytica
<220>
   <221> gene
   <222> (1)..(1146)
   <223> FADH gene
<400> 9
   atgtctaccg aaggaaagac catcacttgc aaggccgccg ttgcctggga ggccggcaag 60
   cccctgaccc ttgaagacgt cgaggttgct cctccccagg cccacgaggt tcgagtcaag 120
   gtcacctaca ctggtgtctg ccacaccgac gcctacactc tcagtggctc tgatcccgag 180
   ggaatcttcc cctctgttct gggccacgag ggtgccggta tcgtcgagtc tatcggcgag 240
   ggcgtcacca gcgtcaagcc cggagactcc gtcattctgc tctacactgc cgagtgcaag 300
   gagtgcaagt tctgcaagtc cggcaagacc aacctgtgcc agaaggtgcg agccacccag 360
   ggtaagggtg ttctgccaga cggaaccacc cggttcaagt gcaagggcaa ggatctctat 420
   tcgtatatgg gctgctcttc cttctcccag tacactgtgg ttgctgacgt ctccctcgtc 480
   gccgtcgacc cctctgctcc ccaggaccga acctgtctgc tcggctgcgg tgtcaccacc 540
   ggctacggag ctgccaccgt caccgccaat gtccagaagg gcgacaatgt tgctgtcttc 600
   ggcgctggct gtgtcggtct ggccgtcgtg atgggcgcca aggagcgagg agccgccaag 660
   atcattgtca ttgacatcaa tggcaacaag gaggcttggg cttacaagtt tggtgccact 720
   gactttgtca accccaccaa gctacccgag ggcaccacta ttgttgacaa gctggtggag 780
   atgaccgacg gaggttgcga cttcaccttt gactgtaccg gaaacgtgac tgtcatgcga 840
   caggctctgg aggcctgcca taagggctgg ggtgagtcga tcatcatcgg cgttgctgct 900
   gctggccagg agattgccac ccgacctttc cagctcgtca ccggccgagt gtggaagggc 960
   tctgcctttg gcggtgtcaa gggccgaacg cagctgcccg agattgtcaa gcgatacaag 1020
   gacggctctt tcgagattga caacttcatc acccactcca agcccctcaa ggacatcaac 1080
   tctgccttca ccgacctgca caagggcgac tgcattcgaa ccgtcgtcga catgtggcag 1140
   gtttag 1146
<210> 10
   <211> 1830
   <212> DNA
   <213> Yarrowia lipolytica
<220>
   <221> gene
   <222> (1)..(1830)
   <223> FAO1 gene
<400> 10
   atgtctgacg acaagcacac tttcgacttt atcattgtcg gtggaggaac cgccggcccc 60
   actctcgccc ggcgactggc cgatgcctgg atctccggta agaagctcaa ggtgctcctg 120
   ctcgagtccg gcccctcttc cgagggtgtt gatgatattc gatgccccgg taactgggtc 180
   aacaccatcc actccgagta cgactggtcc tacgaggtcg acgagcctta cctgtctact 240
   gatggcgagg agcgacgact ctgtggtatc ccccgaggcc attgtctggg tggatcctct 300
   tgtctgaaca cctctttcgt catccgagga acccgaggtg atttcgaccg aatcgaagag 360
   gagaccggcg ctaagggctg gggttgggat gatctgttcc cctacttccg aaagcacgag 420
   tgttacgtgc cccagggatc tgcccacgag cccaagctca ttgacttcga cacctacgac 480
   tacaagaagt tccacggtga ctctggtcct atcaaggtcc agccttacga ctacgcgccc 540
   atctccaaga agttctctga gtctctggct tctttcggct acccttataa ccccgagatc 600
   ttcgtcaacg gaggagcccc ccagggttgg ggtcacgttg ttcgttccac ctccaacggt 660
   gttcgatcca ccggctacga cgctcttgtc cacgccccca agaacctcga cattgtgact 720
   ggccacgctg tcaccaagat tctctttgag aagatcggtg gcaagcagac cgccgttggt 780
   gtcgagacct acaaccgagc tgccgaggag gctggcccta cctacaaggc ccgatacgag 840
   gtggttgtgt gctgcggctc ttatgcctct ccccagcttc tgatggtttc cggtgttgga 900
   cccaagaagg agctcgagga ggttggtgtc aaggacatca ttttggactc tccttacgtt 960
   ggaaagaacc tgcaggacca tcttatctgc ggtatctttg tcgaaattaa ggagcccgga 1020
   tacacccgag accaccagtt cttcgacgac gagggactcg acaagtccac cgaggagtgg 1080
   aagaccaagc gaaccggttt cttctccaat cctccccagg gcattttctc ttacggccga 1140
   atcgacaacc tgctcaagga tgatcccgtc tggaaggagg cctgcgagaa gcagaaggct 1200
   ctcaaccctc gacgagaccc catgggtaac gatccctctc agccccattt cgagatctgg 1260
   aatgctgagc tctacatcga gctagagatg acccaggctc ccgacgaggg ccagtccgtc 1320
   atgaccgtca tcggtgagat tcttcctcct cgatccaagg gttacgtcaa gctgctgtcc 1380
   cccgacccta tggagaaccc cgagattgtc cacaactacc tgcaggaccc tgttgacgct 1440
   cgagtcttcg ctgccatcat gaagcacgcc gccgacgttg ccaccaacgg tgctggcacc 1500
   aaggacctcg tcaaggctcg atggcccccg gagtccaagc ccttcgagga aatgtccatc 1560
   gaggaatggg agacttacgt ccgagacaag tctcacacct gtttccaccc ctgtggtact 1620
   gtcaagcttg gtggtgctaa tgataaggag gccgttgttg acgagcgact ccgagtcaag 1680
   ggtgtcgacg gcctgcgagt tgccgacgtc tctgtccttc cccgagtccc caacggacac 1740
   acccaggctt ttgcctacgc tgttggtgag aaggctgccg acctcatcct tgccgacatt 1800
   gctggaaagg atctccgacc tcgaatctaa 1830
<210> 11
   <211> 1602
   <212> DNA
   <213> Yarrowia lipolytica
<220>
   <221> gene
   <222> (1)..(1602)
   <223> FALDH1 gene
<400> 11
   atgtcctggg aaacaatcac tcctcctacg ccaatcgata cgtttgacag caacttgcaa      60
   cgtcttcgag actctttcga gaccggcaag ctcgactctg tcgactaccg tctcgagcag     120
   ctgcgaaccc tgtggttcaa gttctacgac aacctcgaca acatctacga ggcggtcacc     180
   aaggatctcc atcgacccag gttcgaaacc gagctcaccg aggtactgtt tgttcgagac     240
   gagttctcca ccgtcatcaa gaacctgcga aagtgggtca aggaagaaaa ggtggagaac     300
   cccggaggcc ccttccagtt tgccaacccc cgaatccgac ccgttcctct gggagtggtg     360
   ctggtcatca ctccctggaa ctaccccgtc atgctcaaca tctcacctgt gattgccgcc     420
   attgctgccg gctgtcccat cgtgctcaag atgtccgagc tgtctcccca cacttccgct     480
   gttcttggcc gaatcttcaa ggaggccctg gaccccggta tcatccaggt tgtttacgga     540
   ggtgtccccg agaccaccgc ccttcttacc cagcattggg acaagatcat gtacaccgga     600
   aacggagccg ttggtcgaat catcgcccag gccgcggtca agaacctgac tcctctagct     660
   cttgagcttg gtggcaagtc acccgtgttc atcacttcca actgcaagag cgttatgacg     720
   gccgctcggc gaatcgtgtg gggcaagttt gtcaacgccg gccagatctg tgtcgctcca     780
   gactacattc tggttgctcc cgaaaaggag gccgagctcg tcgcttgtat caaggaggtg     840
   ctccaagaac gatacggctc caagagagac gcccaccacc ccgatctgtc ccatatcatt     900
   tccaagcccc attggaagcg tattcacaac atgatcgccc agaccaaggg agacatccag     960
   gtgggtggac tcgagaacgc cgacgaagac caaaagttca tccagcccac aatcgtctcc    1020
   aacgttccag atgacgacat tctcatgcag gacgagattt tcggacccat catccccatc    1080
   atcaagcccc gaaccctcgg ccagcaggtt gattacgtca caagaaacca tgacaccccc    1140
   ctggccatgt acatcttctc tgacgacccc aaggaggtgg actggctaca gacccgaatc    1200
   cgagctggtt ctgtaaacat caacgaggtc attgagcagg tcggactggc ctctctgcct    1260
   ctcagtggag ttggagcttc cggaaccgga gcataccatg gaaaattctc cttcgatgtc    1320
   ttcacccaca agcaggccgt tatgggacag cccacctggc ccttctttga atacctcatg    1380
   tattaccggt accctcctta ctccgagtac aagatgaagg tgctccgaac cctgttccca    1440
   ccggttctga ttcctcgaac cggccgaccc gacgctactg ttcttcagcg agttctcggc    1500
   aacaagctgc tttggatcat tattgccgcc cttgttgcgt acgccaaacg aaatgagctg    1560
   ctcatcacca ttgctcagat tatgtcggtg tttattaagt ag                        1602
<210> 12
   <211> 1566
   <212> DNA
   <213> Yarrowia lipolytica
<220>
   <221> gene
   <222> (1)..(1566)
   <223> FALDH2 gene
<400> 12
   atgtcagagt tcgattggga gtcaattttg ccggcaacac cactaggtga gatcgagaag      60
   gatattcaaa ccctacgaca gggcttcagg tccggaaaga cgctggattt gaacttcagg     120
   cttgaccaga ttcgtaagct tttctatgct ctctatgata atgtcgatgc gatcaaagaa     180
   gcaattcata aggatctcgg acgtccggtc ttcgagactg aactttgcga gatctccttt     240
   cagtggggtg aattcaataa tgtcgtttct aacttgaaga aatgggcagc tgatgagacg     300
   gtgaagggaa ccaccattca atacactctc acccggccaa agattagaaa gcgtccactt     360
   ggtaccgtcc ttatcatatc tccttggaac tacccatttg ttctgaccat ctctcccctg     420
   cttgctgctc tagcggcagg aaatacggtg gccctaaagt tctccgaaat gtgcccacat     480
   acatcgcttt tgctgggaaa gttgtgcaca gaggcacttg ataaagaaat tttcaaggca     540
   tttcagggag gcgttccggt agtgtcggag attctcaagt acaagttcga caaaatcatg     600
   tacactggaa atcatcgagt tggcaagatc atcttggacg cagctaacaa atacctcacc     660
   cccgttattt tggagcttgg aggcaaatca ccagtcttcg tgactaagaa ttgccaaaac     720
   gtatctcttg ctgccaagcg tgctctgtgg ggtaaactgg tcaacgctgg acaaacatgc     780
   gttgcccccg attacatcat cgtcgagcct gaggtcgaac aggagtttat caaagcttgc     840
   cagtactggg ttgagaagtt ctaccgaggt ggagttgact ctgatcataa ggacttcact     900
   catattgcaa cacctggaca ttggagacga ttgacatcca tgcttgccca gacagaggga     960
   aatatcatca caggcggaaa ttcggacgag aaatcacggt ttcttgctcc cacagttgtt    1020
   gcgaaagttc ctgatggtga ttctttgatg aatgatgaga tctttggccc tatcctgccc    1080
   atcctgacag ccagatccgt tgacgaaggt attcgctatg ttcatgagaa tcacgacact    1140
   cccctggcca tgtatgtctt tactgataat gcatcagaag gagagtatat ccaatctcaa    1200
   atcaactcag gtggcctgat attcaatgat agtcttgttc acgttggctg cgtgcaggcg    1260
   ccttttggtg gtgtcggcca atccggctat gggtcttatc acggcgaaga ttccttcttg    1320
   gctttttcac acaggcagac tttcatgaag cagccccatt tcatcgaacg accaatggcg    1380
   atcagatatg ccccctacac tagtcgaaaa caaaaggctg tccagggtag tctagctgct    1440
   ccatcttttc ctcgaacagg aaaggttgac cgctccctgt tggagcggat atttggtaag    1500
   ctatggttct gggtgatcgt tttagggcta ggagcagcca gtttgaagtc aggaattttc    1560
   ttatga                                                               1566
<210> 13
   <211> 1590
   <212> DNA
   <213> Yarrowia lipolytica
<220>
   <221> gene
   <222> (1)..(1590)
   <223> FALDH3 gene
<400> 13
   atgactacca ctgccacaga gacccccacg acaaacgtga cccccaccac gtcactgccc 60
   aaggagaccg cctccccagg agggaccgct tctgtcaaca cgtcattcga ctgggagagc 120
   atctgcggca agacgccgtt ggaggagatc gagtcggaca tttcgcgtct caaaaagacc 180
   ttccgatcgg gcaaaactct ggatctggac taccgactcg accagatccg aaacctggcg 240
   tatgcgatcc gcgataacga aaacaagatc cgcgacgcca tcaaggcgga cctgaaacga 300
   cctgacttcg aaaccatggc ggccgagttc tcggtccaga tgggcgaatt caactacgtg 360
   gtcaaaaacc tgccgaaatg ggtcaaggac gaaaaagtca agggaaccag catggcgtac 420
   tggaactcgt cgccaaagat ccggaaacgg cccctgggct ccgtgcttgt catcacgccc 480
   tggaactacc cactgattct ggccgtgtcg cctgttctgg gcgccattgc cgcaggcaac 540
   accgtggcgc tgaaaatgtc agaaatgtca cccaacgcgt caaaggtgat tggcgacatt 600
   atgacagctg ccctggaccc ccagctcttt caatgcttct tcggaggagt ccccgaaacc 660
   accgagatcc tcaaacacag atgggacaag atcatgtaca ccggaaacgg caaagtgggc 720
   cgaatcatct gtgaggctgc caacaagtac ttgacacctg tggagctcga actcggagga 780
   aagtcgcctg ttttcgtcac caaacactgc tccaacctgg aaatggccgc ccgccgaatc 840
   atctggggca aattcgtcaa cggaggacaa acctgcgtgg ctccagacta cgttctggtg 900
   tgtcccgagg tccacgacaa atttgtggct gcctgtcaaa aggtgctgga caagttctac 960
   cctaacaact ctgccgagtc cgagatggcc catatcgcca cccctctcca ttacgagcgt 1020
   ttgacgggcc tgctcaattc cacccgaggt aaggtcgttg ctggaggcac tttcaactcg 1080
   gccacccggt tcattgctcc tacgattgtc gacggagtgg atgccaacga ttctctgatg 1140
   cagggagaac tgtttggtcc tcttctcccc attgtcaagg ccatgagcac cgaggctgcc 1200
   tgcaactttg tgcttgagca ccaccccacc cccctggcag agtacatctt ttcagataac 1260
   aattctgaga ttgattacat ccgagatcga gtgtcgtctg gaggtctcgt gatcaacgac 1320
   actctgatcc acgtgggatg cgtacaggcg ccctttggag gtgtcggaga cagtggaaat 1380
   ggaggatacc atggcaagca cactttcgat ttgttcagcc attctcagac ggtcctcaga 1440
   caacccggat gggtcgaaat gctgcagaag aaacggtatc ctccgtacaa caagagcaac 1500
   gagaagtttg tccggagaat ggtggtcccc agccctggtt ttccccggga gggtgacgtg 1560
   agaggatttt ggtcgagact cttcaactag 1590
<210> 14
   <211> 1560
   <212> DNA
   <213> Yarrowia lipolytica
<220>
   <221> gene
   <222> (1)..(1560)
   <223> FALDH4 gene
<400> 14
   atgtctacct ttgattggga atccattgtg cctgccactc ctctcgacca gattcctggc      60
   gacatccagc gactgcgaaa gggcttccga tccggaaaga ccctcgatct caactaccga     120
   ctggaccaga ttcgaaactt gcactacgtc ctcagagaca atgtcgaggc catcaaggac     180
   gccgtgtaca aggatctcgg ccgacccaag cacgagactg acctgtgcga ggtgggtttc     240
   ctgtggggcg agtttaacaa cgtggttgcc aacctcaaga agtgggccgc cgacgaggac     300
   gtcaagacca acctgcagta ctccatctcc tcccccaaga tccgaaagcg acctcttgga     360
   aacgtgctca tcatctcgcc ctggaactac ccctttatgc tgaccgtgtc tcctctcatt     420
   ggagctctgg ctgccggtaa cactgtggct gtcaagttct ccgaaatggc cccccacact     480
   tccaaaattg ttggcgactt gtgcaccaag gccctcgacc ccgacgtctt ccaggccatc     540
   cagggaggtg tccccgtcgt caccaagacc ctcgagcaga agttcgacaa gattatgtac     600
   actggtaacc acactgtcgg taagatcatt gccactgccg ccaacaagta cctgacaccc     660
   gtcatcctcg agctcggagg taagtcgccc gtttttgtca ccaagaactg caagaacatc     720
   aagcttgccg ctaagcgagc cctgtggggt aaggtggtaa acgctggcca gacctgtgtg     780
   gctcccgact acgtgattgt cgagcccgag gtggagcagg agtttatcga cgcctgcaag     840
   tactggatta acgagttcta cagtggtaag attgaccagt acaaccccga ctttgccaag     900
   atcgccaccc ccaaccactg gaaccgactt acctccatgt tgagcaagtc caagggagag     960
   atcattactg gaggtaacac tgacgagaag actcgattca tcgctcctac tgtcgtcgca    1020
   aaggtccccg acaatgattc cctgatggag gacgagattt tcggccctct tctgcccatt    1080
   ctcactgccc gatccgtcga ggagggtatc aagtacgtgc acgagaacca cgacacccct    1140
   cttgccatgt acgtcttcac tgacaaggcc tctgagggcg actacatcca gtcccagatc    1200
   aactctggtg gccttatctt caatgacact ctgatccacg ttggatgtgt ccaggctccg    1260
   tttggtggtg tcggcatgtc cggttacggt gcttaccatg gcgaggactc cttcctggcc    1320
   ttcacccacc gacaaaccta cctcaaccag cccaagcttc tggagcctct tcaggacgtg    1380
   cgatacgccc cctacaccaa aaccaagcga agcatggtca agaacctgct gctggtcggc    1440
   cccattttcc cccgaaccgg ctccgtatac cccaacgtgc tgatccgaat cttccgaaag    1500
   atttggttct gggtccttat tgtcgccatc ggagctgctg gtgccaaggc tctgctctag    1560
<210> 15
   <211> 2034
   <212> DNA
   <213> Yarrowia lipolytica
<220>
   <221> gene
   <222> (1)..(2034)
   <223> ACO1 gene
<400> 15
   atggccaagg agcgaggtaa gactcaattc actgtccgag atgtgaccaa cttcctcaat        60
   ggtggagaag aagagaccca gattgtcgag aagatcatga gcagtattga acgtgatcca       120
   gtactgtctg tcactgctga ctacgactgc aaccttcagc aggcccgaaa acagaccatg       180
   gagcgggtgg ctgctctgtc gccttatctg gtcaccgata ctgagaagct atctctgtgg       240
   cgtgcgcaac tgcatggaat ggttgatatg tctactcgta cgcggttgtc gatccacaac       300
   aacctgttca ttggttccat caggggatct ggtactcctg aacagttcaa gtactgggtc       360
   aagaagggag cggtggctgt taagcagttc tatggatgct ttgccatgac agagttgggc       420
   catggaagca acctcaaggg actagagaca accgccactt atgaccagga cagtgaccag       480
   ttcattatca acactcctca tattggtgct accaagtggt ggattggcgg tgcagcccac       540
   acttccaccc attgtgtttg tttcgcgaaa ctgattgtgc atggcaagga ctatggtact       600
   cgaaactttg tggtacctct ccgaaatgtc cacgatcaca gtctcaaggt cggtgtttca       660
   attggagaca ttggaaagaa gatgggcaga gatggtgttg acaatggctg gatccagttc       720
   accaatgttc gaatccccag acagaacatg ctaatgagat atgccaaggt gtctgatact       780
   ggagtggtaa ccaaacccgc tcttgaccaa ctcacttatg gagccctcat tcgaggtcga       840
   gtgtccatga ttgccgactc gttccacgtc tccaaacgat tcctcacaat tgctcttcgg       900
   tacgcttgtg tccgacgaca gtttggaacc tctggagaca ctaaggagac caagatcatc       960
   gactaccctt accaccagcg acgattgctg cctcttctgg cctactgcta cgctatgaag      1020
   atgggtgctg atgaggctca gaagacttgg attgagacca ccgatcgaat tctggctctc      1080
   aatcccaacg accccgccca gaagaacgat ctggagaagg ccgtcaccga cacaaaggag      1140
   ctgtttgctg cgtctgcagg aatgaaggca tttaccacgt ggggatgtgc caaaatcatt      1200
   gatgagtgcc gacaggcctg tggaggtcat ggatactctg gatataacgg atttggccag      1260
   ggctacgctg actgggttgt ccagtgtacc tgggaaggag acaacaacgt tctgtgtctg      1320
   tcaatgggcc gagggctggt tcagtcagct ctacagattt tggctggaaa gcacgtcggt      1380
   gcttctattc agtacgtagg agacaagtct aaaatctccc agaacggcca gggtaccccc      1440
   agagagcaac ttctgtcccc cgagtttcta gtagaagctt tcagaacggc ttctcgaaac      1500
   aacattctca gaaccaccga taaataccaa gagcttgtca aaactctcaa tcccgaccag      1560
   gcctttgagg agctgtctca gcagagattc cagtgtgctc gaatccacac acgacagcat      1620
   cttatctctt cattctatgc ccgaattgcc actgccaaag acgatatcaa gccccatctg      1680
   ctgaaactgg ccaatctgtt tgccctctgg tcaattgagg aggacactgg aatcttcctg      1740
   cgggagaaca tcctcacccc tggagacatt gacctgatca acagtcttgt ggacgagctc      1800
   tgtgttgcag ttcgagatca ggtaattgga ctcactgatg cctttggtct ctctgacttc      1860
   ttcattaacg ctcccatcgg ctcctacgat ggtaatgttt acgaaaagta ctttgccaag      1920
   gtcaaccagc aaaaccccgc tactaaccct cgtcctccct actacgagtc gactctcaag      1980
   cccttcttgt tccgagaaga ggaggacgat gaaatttgcg atctcgatga gtga            2034
<210> 16
   <211> 2103
   <212> DNA
   <213> Yarrowia lipolytica
<220>
   <221> gene
   <222> (1)..(2103)
   <223> ACO2 gene
<400> 16
   atgaacccca acaacactgg caccattgaa atcaacggta aggagtacaa caccttcacc      60
   gagccccccg tggccatggc tcaggagcga gccaagacct ccttccccgt gcgagagatg       120
   acctacttcc tcgacggtgg cgagaagaac accctcaaaa acgagcagat catggaggag      180
   attgagcgag accctctttt caacaacgac aactactacg atctcaacaa ggagcagatc      240
   cgagagctca ccatggagcg agtcgccaag ctgtctctgt ttgtgcgtga tcagcccgag      300
   gacgacatca agaagcgatt tgctctcatt ggtatcgccg atatgggaac ctacacccga      360
   cttggtgtcc actacggcct cttctttggc gccgtccgag gtaccggaac tgccgagcag      420
   tttggccact ggatctccaa gggagccgga gacctgcgaa agttctacgg atgtttctcc      480
   atgaccgagc tgggccatgg ctccaacctg gctggtctcg agaccaccgc catctacgat      540
   gaggagaccg acgagttcat catcaacacc cctcacattg ccgccaccaa gtggtggatt      600
   ggaggagccg cccacaccgc cacccacact gtcgtgttcg cccgactcat tgtcaagggc      660
   aaggactacg gtgtcaagac ctttgttgtc cagctgcgaa acatcaacga ccacagcctc      720
   aaggtcggta tctctattgg tgatatcgga aagaagatgg gccgagacgg tatcgataac      780
   ggatggatcc agttcaccaa cgtgcgaatc cccgacagaa acctgctcat gaagtacaca       840
   aaggtcgacc gagagggtaa cgtgacccag cctcctctgg ctcagcttac ctacggttct      900
   cttatcactg gtcgagtctc catggcctct gattctcacc aggtcggaaa gcgattcatc 960
   accattgctc tgcgatacgc ctgcattcga cgacagttct ccaccacccc cggccagccc 1020
   gagaccaaga tcatcgacta cccctaccat cagcgacgac ttctgcctct tctggcctat 1080
   gtctatgctc ttaagatgac tgccgatgag gttggagctc tcttctcccg aaccatgctt 1140
   aagatggacg acctcaagcc cgacgacaag gccggcctca atgaggttgt ttccgacgtc 1200
   aaggagctct tctccgtctc cgccggtctc aaggccttct ccacctgggc ttgtgccgac 1260
   gtcattgaca agacccgaca ggcttgcggt ggccacggtt actctggata caacggtttc 1320
   ggccaggcct acgccgactg ggttgtccag tgcacctggg agggtgacaa caacattctc 1380
   accctttctg ccggccgagc tcttatccag tctgccgttg ctctgcgaaa gggcgagcct 1440
   gttggtaacg ccgtttctta cctgaagcga tacaaggatc tggccaacgc taagctcaat 1500
   ggccgatctc tcaccgaccc caaggtcctc gtcgaggcct gggaggttgc tgccggtaac 1560
   atcatcaacc gagccaccga ccagtacgag aagctcattg gcgagggtct taacgccgac 1620
   caggcctttg aggttctgtc tcagcagcga ttccaggccg ccaaggtcca cacacgacga 1680
   cacctcattg ccgctttctt ctcccgaatt gacaccgagg ctggcgaggc catcaagcag 1740
   cccctgctta acctggctct gctgtttgcc ctgtggtcca tcgaagagga ctctggtctg 1800
   ttcctgcgag agggcttcct cgagcccaag gatatcgaca ccgtcaccga gctcgtcaac 1860
   aagtactgca ccactgtgcg agaggaggtc attggctaca ccgatgcctt caacctgtcc 1920
   gactacttca tcaacgctcc tattggatgc tacgatggtg acgcttaccg acactacttc 1980
   cagaaggtca acgagcagaa ccctgcccga gacccccgac ctccttacta cgcctctact 2040
   ctcaagccct tccttttccg agaggaggag gatgatgaca tttgcgagct tgatgaggaa 2100
   tag 2103
<210> 17
   <211> 2103
   <212> DNA
   <213> Yarrowia lipolytica
<220>
   <221> gene
   <222> (1)..(2103)
   <223> ACO3 gene
<400> 17
   atgatctccc ccaacctcac agctaacgtc gagattgacg gcaagcagta caacaccttc      60
   acagagccac ccaaggcgct cgccggcgag cgagccaagg tcaagttccc catcaaggac     120
   atgacggagt ttctgcacgg tggcgaggag aacgtgacca tgatcgagcg actgatgacg     180
   gagctcgagc gagaccccgt gctcaacgtg tcgggcgact acgacatgcc caaggagcag     240
   ctgcgagaga cggccgtggc gcgaattgcg gcgctgtccg gccactggaa gaaggacaca 300
   gaaaaggagg cgctgctgcg gtcccagctg cacggcattg tggacatggg cacccgaatc 360
   cgactcggtg tgcacacggg cctgttcatg ggcgccatcc ggggttccgg caccaaggag 420
   cagtacgact actgggtgcg aaagggcgcc gcggacgtca agggcttcta cggctgcttt 480
   gctatgaccg agctgggcca tggctccaac gtggccggtc ttgagaccac cgccacctac 540
   atccaggaca cggacgagtt catcatcaac acccccaaca ctggagccac caagtggtgg 600
   attggaggag ccgcccactc ggccacccac accgcctgct ttgctcgtct gcttgtcgac 660
   ggcaaggact acggcgtcaa gatctttgtt gtccagctgc gagacgtctc ttctcactct 720
   ctcatgcccg gcatcgctct cggcgacatt ggaaagaaga tgggccgaga cgccatcgac 780
   aacggctgga tccagttcac caatgtgcga atcccccgac agaacatgct catgaagtac 840
   gccaaggtct cgtctaccgg caaggtgtcg cagcctcctc tggcccagct cacctacggc 900
   gctctcattg gcggccgagt caccatgatt gccgactcct tctttgtctc ccagcgattc 960
   atcaccattg ctctgcgata cgcctgtgtg cgacgacagt ttggcaccac ccccggccag 1020
   cccgagacta agatcatcga ctacccctac catcagcgac gtctgctgcc tcttctggcc 1080
   ttcacctacg ccatgaagat ggccgccgac cagtcccaga ttcagtacga tcagaccacc 1140
   gatctgctgc agaccatcga ccctaaggac aagggcgctc tgggcaaggc cattgtcgac 1200
   ctcaaggagc tgtttgcctc ttctgctggt ctcaaggcct tcaccacctg gacctgtgcc 1260
   aacatcattg accagtgccg acaggcctgc ggtggccacg gctactctgg ctacaacggc 1320
   tttggccagg cctacgccga ctgggttgtc cagtgcacct gggagggtga caacaacgtc 1380
   ctgtgtctgt ccatgggccg aggtctcatc cagtcgtgtc tgggccaccg aaagggtaag 1440
   cctctgggct cttctgtcgg ctacctggct aacaagggtc ttgagcaggc tactctgagc 1500
   ggccgagacc tcaaggaccc caaggttctc atcgaggcct gggagaaggt cgccaacggc 1560
   gccatccagc gggccactga caaatttgtc gagctcacca agggcggcct ctctcctgac 1620
   caggcctttg aggagctgtc gcagcagcga ttccagtgtg ccaagatcca cacccgaaag 1680
   cacctggtga ctgccttcta cgagcgaatc aacgcctctg cgaaggccga cgtcaagcct 1740
   tacctcatca acctcgccaa cctcttcact ctgtggtcca ttgaggagga ctctggtctc 1800
   ttcctgcgag agggtttcct gcagcccaag gacattgacc aggtgactga gctggtgaac 1860
   cactactgca aggaggttcg agaccaggtt gccggctaca ccgatgcctt tggtctgtct 1920
   gactggttca tcaacgctcc cattggaaac tacgatggtg acgtttacaa gcattacttt 1980
   gccaaggtta accagcagaa ccctgctcag aacccccgac ctccttacta tgagagcact 2040
   cttcgacctt tcctgttccg agaggatgag gatgacgaca tttgcgagct ggacgaggaa 2100
   tag 2103
<210> 18
   <211> 2106
   <212> DNA
   <213> Yarrowia lipolytica
<220>
   <221> gene
   <222> (1)..(2106)
   <223> ACO4 gene
<400> 18
   atgatcaccc caaaccccgc taacgacatt gtccatgacg gcaagctcta cgacaccttc 60
   actgagcccc ccaagctgat ggctcaggag cgagctcagc tggacttcga ccctagagac 120
   atcacctact ttctggatgg ctctaaggag gagaccgagc tgctggagtc gctcatgctc 180
   atgtacgagc gagaccctct cttcaacaac cagaacgagt acgatgaatc gtttgaaaca 240
   ctgcgagagc gatctgtgaa gcgaattttc cagctgtcca agtccatcgc catggacccc 300
   gagcccatgt ctttccgaaa gattgggttc ctgggtattc ttgacatggg aacgtatgct 360
   cgactgggag tccactacgc gctcttctgt aactccatcc ggggccaggg aacccccgat 420
   cagctcatgt actggctgga ccagggagcc atggtcatca agggcttcta cggctgtttt 480
   gccatgaccg aaatgggcca tggatctaac ctgtcgcgtc tggaaaccat cgccactttc 540
   gacaaagaga ccgacgaatt tatcattaac acgccccacg ttggagccac aaagtggtgg 600
   attggtggtg ctgctcacac tgctactcac acacttgcct ttgcccgtct tcaagtagac 660
   ggaaaggact acggtgtgaa atcgtttgtc gtacctctcc gaaacctgga cgaccattcg 720
   ctgcgtcctg gaatcgccac aggtgatatt ggtaagaaga tgggtcgaga tgccgttgac 780
   aacggctgga ttcagttcac caacgtccga gtgccccgaa actacatgct catgaagcat 840
   accaaggttc ttcgagacgg taccgtcaag cagccgcctt tggcccaact gacttacgga 900
   tctctcatca ctggacgagt ccagatgacc actgactctc acaatgtgtc caaaaagttc 960
   ctcaccattg ccctgagata cgccaccatc cgacgacagt tctcgtcaac tccaggagag 1020
   cccgaaaccc gactaattga ctacctgtac caccaaagac gactcctgcc tcttatggct 1080
   tactcttacg ccatgaaact agctggagat cacgtccgag agctgttctt tgcatcccag 1140
   gagaaggctg agagcctcaa ggaggacgac aaagccggag ttgagtctta cgtccaggat 1200
   atcaaggagc tcttctctgt ttctgctggt ctcaaggctg ccactacatg ggcttgtgct 1260
   gacatcattg acaaggcccg acaggcgtgt ggaggccacg gatactctgc ctacaacggc 1320
   tttggacagg ccttccagga ctgggttgtc cagtgcactt gggagggtga caatactgtt 1380
   ctgactctat ctgccggccg agctctgatc caatctgctc tcgtctaccg aaaggagggc 1440
   aaactaggta acgccacgaa gtacctctct cggtccaagg agcttgccaa cgccaagaga 1500
   aacggacgat ccctggaaga ccccaagctg ctcgtggagg catgggaggc tgtctctgcc 1560
   ggtgctatca acgctgctac tgacgcttac gaggagctct ccaagcaggg agtttctgtt 1620
   gacgagtgct ttgagcaggt gtcccaggag cgattccagg ctgcccgaat ccacactcga 1680
   cgagctctta tcgaggcctt ctactcacga atcgccactg ctgatgagaa ggtgaagcct 1740
   catctgatcc ctctggccaa cctgtttgcc ctgtggtcca ttgaggagga ctctgctctg 1800
   ttcctggctg agggctactt tgagcctgag gatatcattg aggtgacttc tcttgtcaac 1860
   aagtactgcg gaattgttcg aaagaacgtt attggataca ccgatgcctt caacctgtcc 1920
   gactacttca tcaacgctgc cattggacga tacgacggag acgtgtacaa gaactacttt 1980
   gagaaggtca aacagcagta ccctcctgag ggtggcaagc ctcactacta cgaggatgtc 2040
   atgaagccct tcctgcatcg agagcgaatt cccgatgtcc ccatggagcc cgaggatatt 2100
   cagtaa 2106
<210> 19
   <211> 2100
   <212> DNA
   <213> Yarrowia lipolytica
<220>
   <221> gene
   <222> (1)..(2100)
   <223> ACO5 gene
<400> 19
<210> 20
   <211> 2070
   <212> DNA
   <213> Yarrowia lipolytica
<220>
   <221> gene
   <222> (1)..(2070)
   <223> ACO6 gene
<400> 20
   atgctctctc aacagtccct caacacgttt accgagcccc cggtcgaaat ggcccgggag 60
   cgaaaccaga cttccttcaa cccgcgtctg ctgacgtact ttctggacgg aggcgaaaag 120
   aacactctgc ttatggaccg actgatgcaa gagtacgagc gagaccctgt gtttcgaaac 180
   gagggcgact acgatattac cgatgtggcc cagtcgcgag agctggcctt caagcgaatc 240
   gccaagctca tcgagtatgt gcacaccgac gacgaggaga cgtatctgta ccgatgcatg 300
   cttctgggcc aaatcgatat gggagccttt gcccggtacg ccatccacca cggagtctgg 360
   ggcggtgcca ttcgaggtgc aggaacgcct gagcagtacg aattctgggt caagaaagga 420
   tctctgtcgg ttaagaagtt ctatggatcc ttctccatga ccgagctggg ccacggcagt 480
   aacttggtgg gtctggagac caccgccacc ctggacaaga acgcagacga gttcgtgatc 540
   aacactccca acgttgctgc cactaaatgg tggatcggag gagccgccga taccgccact 600
   cacacagctg tgtttgcacg tctcattgtc gacggagagg accacggtgt caagacgttt 660
   gtggtgcagc tgcgagacgt ggagactcac aacctgatgc ctggtattgc tatcggagac 720
   tgcggcaaga agatgggacg tcagggaacc gacaacggct ggatccagtt cacccatgtg 780
   cgaattcccc gacagaacat gctcatgcga tactgtcacg tggacagcga cggaaatgtt 840
   accgagccca tgatggctca gatggcctac ggagctcttc tggctggccg agtcggaatg 900
   gccatggaca gttatttcac ctcgcgaaag ttccttacca ttgctcttcg atatgccacc 960
   attcgacgag cttttgctgc cggaggaggt caggagacca agctgatcga ctacccttac 1020
   caccagcgac gtctgctccc cctcatggcc cagacatatg ccatcaagtg caccgccgat 1080
   aaggtcagag atcagttcgt caaggtcacc gacatgctcc taaacctcga tgtttctgac 1140
   caagaggccg tgcccaaggc cattgccgag gctaaggagc tcttctctgt ttctgctggt 1200
   gtcaaggcta ccacaacttg ggcttgcgca cacaccattg accagtgcag acaggcgtgt 1260
   ggaggccacg gatactctgc ttacaacggt tttggacgtg cttactccga ttgggtgatc 1320
   cagtgcacct gggagggaga caataacatt ctgtgtctgt cagctggcag agctctggtc 1380
   cagtctaacc gagctgtccg ggctggcaag cccattggag gtcctaccgc ctacctggct 1440
   gctcccgctg gttcccccaa gctcgctggt cgaaacttgt acgaccccaa ggtcatgatt 1500
   ggggcctggg agactgtttc ccgagctctg atcaaccgaa ccaccgatga gtttgaggtg 1560
   ctggccaaga agggtctgtc tactgcccag gcctacgagg agctgtccca gcaacgattc 1620
   ctgtgtactc gaatccacac ccgtctgtac atggtcaaga acttctacga gcgaattgcc 1680
   gaggagggca ccgagttcac caaggagcct cttaccagac ttgccaacct gtacgccttc 1740
   tggtccgtcg aagaggaggc tggaatcttc ctccgagagg gctacatcac tccccaggag 1800
   ctcaagtaca tcagtgccga gatccgaaag cagctcttgg aggtgcgaaa ggacgtcatt 1860
   ggctacaccg atgccttcaa cgtgcctgat tttttcctca actctgccat tggacgagct 1920
   gacggagatg tctacaagaa ctacttcaag gtggtcaaca ctcagaaccc tccccaagac 1980
   cctcgacctc cttattacga gtctgtcatt agacccttcc tgttccgaaa ggacgaggat 2040
   gaggaaattt gctctcttga ggatgagtag 2070
<210> 21
   <211> 1205
   <212> DNA
   <213> Yarrowia lipolytica
<220>
   <221> gene
   <222> (1)..(1205)
   <223> Ura3 gene
<400> 21
   cgcccagaga gccattgacg ttctttctaa tttggaccga tagccgtata gtccagtcta 60
   tctataagtt caactaactc gtaactatta ccataacata tacttcactg ccccagataa 120
   ggttccgata aaaagttctg cagactaaat ttatttcagt ctcctcttca ccaccaaaat 180
   gccctcctac gaagctcgag ctaacgtcca caagtccgcc tttgccgctc gagtgctcaa 240
   gctcgtggca gccaagaaaa ccaacctgtg tgcttctctg gatgttacca ccaccaagga 300
   gctcattgag cttgccgata aggtcggacc ttatgtgtgc atgatcaaaa cccatatcga 360
   catcattgac gacttcacct acgccggcac tgtgctcccc ctcaaggaac ttgctcttaa 420
   gcacggtttc ttcctgttcg aggacagaaa gttcgcagat attggcaaca ctgtcaagca 480
   ccagtaccgg tgtcaccgaa tcgccgagtg gtccgatatc accaacgccc acggtgtacc 540
   cggaaccgga atcattgctg gcctgcgagc tggtgccgag gaaactgtct ctgaacagaa 600
   gaaggaggac gtctctgact acgagaactc ccagtacaag gagttcctag tcccctctcc 660
   caacgagaag ctggccagag gtctgctcat gctggccgag ctgtcttgca agggctctct 720
   ggccactggc gagtactcca agcagaccat tgagcttgcc cgatccgacc ccgagtttgt 780
   ggttggcttc attgcccaga accgacctaa gggcgactct gaggactggc ttattctgac 840
   ccccggggtg ggtcttgacg acaagggaga cgctctcgga cagcagtacc gaactgttga 900
   ggatgtcatg tctaccggaa cggatatcat aattgtcggc cgaggtctgt acggccagaa 960
   ccgagatcct attgaggagg ccaagcgata ccagaaggct ggctgggagg cttaccagaa 1020
   gattaactgt tagaggttag actatggata tgtaatttaa ctgtgtatat agagagcgtg 1080
   caagtatgga gcgcttgttc agcttgtatg atggtcagac gacctgtctg atcgagtatg 1140
   tatgatactg cacaacctgt gtatccgcat gatctgtcca atggggcatg ttgttgtgtt 1200
   tctcg 1205
<210> 22
   <211> 36
   <212> DNA
   <213> Artificial Sequence
<220>
   <223> BglII F primer for HisG1
<400> 22
   aattgggccc agatctcaga ccggttcaga caggat 36
<210> 23
   <211> 36
   <212> DNA
   <213> Artificial Sequence
<220>
   <223> EcoRI R primer for HisG1
<400> 23
   tctctgggcg gaattcggag gtgcggatat gaggta 36
<210> 24
   <211> 37
   <212> DNA
   <213> Artificial Sequence
<220>
   <223> NotI F primer for HisG1
<400> 24
   tgtttctcgg cggccgccag accggttcag acaggat 37
<210> 25
   <211> 36
   <212> DNA
   <213> Artificial Sequence
<220>
   <223> BamHI R primer for HisG1
<400> 25
   tccaacgcgt ggatccggag gtgcggatat gaggta 36
<210> 26
   <211> 36
   <212> DNA
   <213> Artificial Sequence
<220>
   <223> BglII F primer for HisG2
<400> 26
   aattgggccc agatctaacg ctacctcgac cagaaa 36
<210> 27
   <211> 36
   <212> DNA
   <213> Artificial Sequence
<220>
   <223> EcoRI R primer for HisG2
<400> 27
   tctctgggcg gaattctctt ctcgatcggc agtacc 36
<210> 28
   <211> 37
   <212> DNA
   <213> Artificial Sequence
<220>
   <223> NotI F primer for HisG2
<400> 28
   tgtttctcgg cggccgcaac gctacctcga ccagaaa 37
<210> 29
   <211> 36
   <212> DNA
   <213> Artificial Sequence
<220>
   <223> BamHI R primer for HisG2
<400> 29
   tccaacgcgt ggatcctctt ctcgatcggc agtacc 36
<210> 30
   <211> 36
   <212> DNA
   <213> Artificial Sequence
<220>
   <223> BglII F primer for glt2
<400> 30
   aattgggccc agatcttcag aacttgcgcc gataaa 36
<210> 31
   <211> 38
   <212> DNA
   <213> Artificial Sequence
<220>
   <223> EcoRI R primer for glt2
<400> 31
   tctctgggcg gaattccttt gccagctaga ccatagag 38
<210> 32
   <211> 37
   <212> DNA
   <213> Artificial Sequence
<220>
   <223> NotI F primer for glt2
<400> 32
   tgtttctcgg cggccgctca gaacttgcgc cgataaa 37
<210> 33
   <211> 38
   <212> DNA
   <213> Artificial Sequence
<220>
   <223> BamHI R primer for glt2
<400> 33
   tccaacgcgt ggatcccttt gccagctaga ccatagag 38
<210> 34
   <211> 35
   <212> DNA
   <213> Artificial Sequence
<220>
   <223> BglII F primer for glt3
<400> 34
   aattgggccc agatctattg gcgggttcgt tactt 35
<210> 35
   <211> 38
   <212> DNA
   <213> Artificial Sequence
<220>
   <223> EcoRI R primer for glt3
<400> 35
   tctctgggcg gaattccctg gaagaaggcc gtattatc 38
<210> 36
   <211> 36
   <212> DNA
   <213> Artificial Sequence
<220>
   <223> NotI F primer for glt3
<400> 36
   tgtttctcgg cggccgcatt ggcgggttcg ttactt 36
<210> 37
   <211> 38
   <212> DNA
   <213> Artificial Sequence
<220>
   <223> BamHI R primer for glt3
<400> 37
   tccaacgcgt ggatcccctg gaagaaggcc gtattatc 38
<210> 38
   <211> 20
   <212> DNA
   <213> Artificial Sequence
<220>
   <223> F1 primer for ACO1
<400> 38
   ttcctcaatg gtggagaaga 20
<210> 39
   <211> 46
   <212> DNA
   <213> Artificial Sequence
<220>
   <223> R1 primer for ACO1
<400> 39
   tctttatcct gtctgaaccg gtctggtacc atagtccttg ccatgc 46
<210> 40
   <211> 45
   <212> DNA
   <213> Artificial Sequence
<220>
   <223> F2 primer for ACO1
<400> 40
   atcgctacct catatccgca cctcccttct gtcccccgag tttct 45
<210> 41
   <211> 19
   <212> DNA
   <213> Artificial Sequence
<220>
   <223> R2 primer for ACO1
<400> 41
   aagaagggct tgagagtcg 19
<210> 42
   <211> 17
   <212> DNA
   <213> Artificial Sequence
<220>
   <223> F1 primer for ACO2
<400> 42
   cccaacaaca ctggcac 17
<210> 43
   <211> 43
   <212> DNA
   <213> Artificial Sequence
<220>
   <223> R1 primer for ACO2
<400> 43
   tctttatcct gtctgaaccg gtctgctcct catcgtagat ggc 43
<210> 44
   <211> 42
   <212> DNA
   <213> Artificial Sequence
<220>
   <223> F2 primer for ACO2
<400> 44
   atcgctacct catatccgca cctccgacaa gacccgacag gc 42
<210> 45
   <211> 18
   <212> DNA
   <213> Artificial Sequence
<220>
   <223> R2 primer for ACO2
<400> 45
   agaccagagt cctcttcg 18
<210> 46
   <211> 19
   <212> DNA
   <213> Artificial Sequence
<220>
   <223> F1 primer for ACO3
<400> 46
   accttcacag agccaccca 19
<210> 47
   <211> 36
   <212> DNA
   <213> Artificial Sequence
<220>
   <223> R1 primer for ACO3
<400> 47
   atggctctct gggcggtgtt gggggtgttg atgatg 36
<210> 48
   <211> 36
   <212> DNA
   <213> Artificial Sequence
<220>
   <223> F2 primer for ACO3
<400> 48
   ttgttgtgtt tctcgcaagg ttctcatcga ggcctg 36
<210> 49
   <211> 22
   <212> DNA
   <213> Artificial Sequence
<220>
   <223> R2 primer for ACO3
<400> 49
   aggaaaggtc gaagagtgct ct 22
<210> 50
   <211> 17
   <212> DNA
   <213> Artificial Sequence
<220>
   <223> F1 primer for ACO4
<400> 50
   actgcgagag cgatctg 17
<210> 51
   <211> 45
   <212> DNA
   <213> Artificial Sequence
<220>
   <223> R1 primer for ACO4
<400> 51
   tctttatcct gtctgaaccg gtctgttcat gagcatgtag tttcg 45
<210> 52
   <211> 44
   <212> DNA
   <213> Artificial Sequence
<220>
   <223> F2 primer for ACO4
<400> 52
   atcgctacct catatccgca cctccgagga cgacaaagcc ggag 44
<210> 53
   <211> 19
   <212> DNA
   <213> Artificial Sequence
<220>
   <223> R2 primer for ACO4
<400> 53
   agagcagagt cctcctcaa 19
<210> 54
   <211> 22
   <212> DNA
   <213> Artificial Sequence
<220>
   <223> F1 primer for ACO5
<400> 54
   aacttcctca caggcagcga gc 22
<210> 55
   <211> 39
   <212> DNA
   <213> Artificial Sequence
<220>
   <223> R1 primer for ACO5
<400> 55
   atggctctct gggcggagta gagagtggga gttgaggtc 39
<210> 56
   <211> 34
   <212> DNA
   <213> Artificial Sequence
<220>
   <223> F2 primer for ACO5
<400> 56
   ttgttgtgtt tctcgccccg tcaaggacgc tgag 34
<210> 57
   <211> 22
   <212> DNA
   <213> Artificial Sequence
<220>
   <223> R2 primer for ACO5
<400> 57
   acagtaaggt ggggcttgac tc 22
<210> 58
   <211> 21
   <212> DNA
   <213> Artificial Sequence
<220>
   <223> F1 primer for ACO6
<400> 58
   agtccctcaa cacgtttacc g 21
<210> 59
   <211> 46
   <212> DNA
   <213> Artificial Sequence
<220>
   <223> R1 primer for ACO6
<400> 59
   tctttatcct gtctgaaccg gtctgccatt tagtggcagc aacgtt 46
<210> 60
   <211> 45
   <212> DNA
   <213> Artificial Sequence
<220>
   <223> F2 primer for ACO6
<400> 60
   atcgctacct catatccgca cctccgagct ctgatcaacc gaacc 45
<210> 61
   <211> 20
   <212> DNA
   <213> Artificial Sequence
<220>
   <223> R2 primer for ACO6
<400> 61
   aggaagggtc taatgacaga 20
<210> 62
   <211> 17
   <212> DNA
   <213> Artificial Sequence
<220>
   <223> F1 primer for FALDH1
<400> 62
   aatcactcct cctacgc 17
<210> 63
   <211> 43
   <212> DNA
   <213> Artificial Sequence
<220>
   <223> R1 primer for FALDH1
<400> 63
   tctttatcct gtctgaaccg gtctgtggtc tcggggacac ctc 43
<210> 64
   <211> 43
   <212> DNA
   <213> Artificial Sequence
<220>
   <223> F2 primer for FALDH1
<400> 64
   atcgctacct catatccgca cctccccatc atcaagcccc gaa 43
<210> 65
   <211> 20
   <212> DNA
   <213> Artificial Sequence
<220>
   <223> R2 primer for FALDH1
<400> 65
   accgacataa tctgagcaat 20
<210> 66
   <211> 19
   <212> DNA
   <213> Artificial Sequence
<220>
   <223> F1 primer for FALDH2
<400> 66
   accactaggt gagatcgag 19
<210> 67
   <211> 45
   <212> DNA
   <213> Artificial Sequence
<220>
   <223> R1 primer for FALDH2
<400> 67
   tctttatcct gtctgaaccg gtctgctccg acactaccgg aacgc 45
<210> 68
   <211> 43
   <212> DNA
   <213> Artificial Sequence
<220>
   <223> F2 primer for FALDH2
<400> 68
   atcgctacct catatccgca cctcccttgc tcccacagtt gtt 43
<210> 69
   <211> 19
   <212> DNA
   <213> Artificial Sequence
<220>
   <223> R2 primer for FALDH2
<400> 69
   gatcacccag aaccatagc 19
<210> 70
   <211> 19
   <212> DNA
   <213> Artificial Sequence
<220>
   <223> F1 primer for FALDH3
<400> 70
   gtgaccccca ccacgtcac 19
<210> 71
   <211> 46
   <212> DNA
   <213> Artificial Sequence
<220>
   <223> R1 primer for FALDH3
<400> 71
   tctttatcct gtctgaaccg gtctgttctg acattttcag cgccac 46
<210> 72
   <211> 45
   <212> DNA
   <213> Artificial Sequence
<220>
   <223> F2 primer for FALDH3
<400> 72
   atcgctacct catatccgca cctccccatt acgagcgttt gacgg 45
<210> 73
   <211> 17
   <212> DNA
   <213> Artificial Sequence
<220>
   <223> R2 primer for FALDH3
<400> 73
   cagggctggg gaccacc 17
<210> 74
   <211> 19
   <212> DNA
   <213> Artificial Sequence
<220>
   <223> F1 primer for FALDH4
<400> 74
   taccgactgg accagattc 19
<210> 75
   <211> 46
   <212> DNA
   <213> Artificial Sequence
<220>
   <223> R1 primer for FALDH4
<400> 75
   tctttatcct gtctgaaccg gtctgcggca gtggcaatga tcttac 46
<210> 76
   <211> 46
   <212> DNA
   <213> Artificial Sequence
<220>
   <223> F2 primer for FALDH4
<400> 76
   atcgctacct catatccgca cctccgactc gattcatcgc tcctac 46
<210> 77
   <211> 21
   <212> DNA
   <213> Artificial Sequence
<220>
   <223> R2 primer for FALDH4
<400> 77
   caaatctttc ggaagattcg g 21
<210> 78
   <211> 20
   <212> DNA
   <213> Artificial Sequence
<220>
   <223> F1 primer for FAO1
<400> 78
   atcattgtcg gtggaggaac 20
<210> 79
   <211> 45
   <212> DNA
   <213> Artificial Sequence
<220>
   <223> R1 primer for FAO1
<400> 79
   acgcctttct ggtcgaggta gcgttgcgta gtcgtaaggc tggac 45
<210> 80
   <211> 45
   <212> DNA
   <213> Artificial Sequence
<220>
   <223> F2 primer for FAO1
<400> 80
   attctggtac tgccgatcga gaagaccgtc atcggtgaga ttctt 45
<210> 81
   <211> 20
   <212> DNA
   <213> Artificial Sequence
<220>
   <223> R2 primer for FAO1
<400> 81
   attcgaggtc ggagatcctt 20
<210> 82
   <211> 20
   <212> DNA
   <213> Artificial Sequence
<220>
   <223> F1 primer for ADH1
<400> 82
   cccagaaggc tgtcattttc 20
<210> 83
   <211> 45
   <212> DNA
   <213> Artificial Sequence
<220>
   <223> R1 primer for ADH1
<400> 83
   acgcctttct ggtcgaggta gcgtttcgca gttcttgggg atatg 45
<210> 84
   <211> 45
   <212> DNA
   <213> Artificial Sequence
<220>
   <223> F2 primer for ADH1
<400> 84
   attctggtac tgccgatcga gaagagccga caaggagaag atgtg 45
<210> 85
   <211> 19
   <212> DNA
   <213> Artificial Sequence
<220>
   <223> R2 primer for ADH1
<400> 85
   caatcttgcc ctcctccat 19
<210> 86
   <211> 20
   <212> DNA
   <213> Artificial Sequence
<220>
   <223> F1 primer for ADH2
<400> 86
   ccagaagggt gtcatcttcg 20
<210> 87
   <211> 45
   <212> DNA
   <213> Artificial Sequence
<220>
   <223> R1 primer for ADH2
<400> 87
   acgcctttct ggtcgaggta gcgttatcgc agttcttggg aatgt 45
<210> 88
   <211> 45
   <212> DNA
   <213> Artificial Sequence
<220>
   <223> F2 primer for ADH2
<400> 88
   attctggtac tgccgatcga gaagaccgac aaggagaaga tgtgc 45
<210> 89
   <211> 20
   <212> DNA
   <213> Artificial Sequence
<220>
   <223> R2 primer for ADH2
<400> 89
   caatcttgcc ctcctccata 20
<210> 90
   <211> 20
   <212> DNA
   <213> Artificial Sequence
<220>
   <223> F1 primer for ADH3
<400> 90
   agaaagccgt catcttcgag 20
<210> 91
   <211> 45
   <212> DNA
   <213> Artificial Sequence
<220>
   <223> R1 primer for ADH3
<400> 91
   ttgcacaagt aacgaacccg ccaattcaca gttcttgggg atgtg 45
<210> 92
   <211> 46
   <212> DNA
   <213> Artificial Sequence
<220>
   <223> F2 primer for ADH3
<400> 92
   ggagataata cggccttctt ccagggctga caaggagaag atgtgc 46
<210> 93
   <211> 20
   <212> DNA
   <213> Artificial Sequence
<220>
   <223> R2 primer for ADH3
<400> 93
   acttggagca gtccagaacg 20
<210> 94
   <211> 20
   <212> DNA
   <213> Artificial Sequence
<220>
   <223> F1 primer for ADH4
<400> 94
   gtcaaaacgt cgacgaacct 20
<210> 95
   <211> 45
   <212> DNA
   <213> Artificial Sequence
<220>
   <223> R1 primer for ADH4
<400> 95
   aggtatttat cggcgcaagt tctgaggctt gaggtcaatg tcgat 45
<210> 96
   <211> 45
   <212> DNA
   <213> Artificial Sequence
<220>
   <223> F2 primer for ADH4
<400> 96
   ctcctctatg gtctagctgg caaaggacat ggaggcccac tctaa 45
<210> 97
   <211> 20
   <212> DNA
   <213> Artificial Sequence
<220>
   <223> R2 primer for ADH4
<400> 97
   agtactccca agcgtcctca 20
<210> 98
   <211> 18
   <212> DNA
   <213> Artificial Sequence
<220>
   <223> F1 primer for ADH5
<400> 98
   gagagccgct ttcaccac 18
<210> 99
   <211> 45
   <212> DNA
   <213> Artificial Sequence
<220>
   <223> R1 primer for ADH5
<400> 99
   aggtatttat cggcgcaagt tctgaagagc ctggtaggca gtgag 45
<210> 100
   <211> 45
   <212> DNA
   <213> Artificial Sequence
<220>
   <223> F2 primer for ADH5
<400> 100
   ctcctctatg gtctagctgg caaagttcca ggacgtgatc aagga 45
<210> 101
   <211> 21
   <212> DNA
   <213> Artificial Sequence
<220>
   <223> R2 primer for ADH5
<400> 101
   taaggatgat cttgccggta g 21
<210> 102
   <211> 20
   <212> DNA
   <213> Artificial Sequence
<220>
   <223> F1 primer for ADH6
<400> 102
   gacccagaaa gccattgtgt 20
<210> 103
   <211> 45
   <212> DNA
   <213> Artificial Sequence
<220>
   <223> R1 primer for ADH6
<400> 103
   aggtatttat cggcgcaagt tctgaagcca cctgagaaag gtctg 45
<210> 104
   <211> 45
   <212> DNA
   <213> Artificial Sequence
<220>
   <223> F2 primer for ADH6
<400> 104
   ctcctctatg gtctagctgg caaagcaccg aggagaagga gaaga 45
<210> 105
   <211> 20
   <212> DNA
   <213> Artificial Sequence
<220>
   <223> R2 primer for ADH6
<400> 105
   tccctcctcc atcaaggtaa 20
<210> 106
   <211> 21
   <212> DNA
   <213> Artificial Sequence
<220>
   <223> F1 primer for ADH7
<400> 106
   gacgttccca agacacaaaa g 21
<210> 107
   <211> 45
   <212> DNA
   <213> Artificial Sequence
<220>
   <223> R1 primer for ADH7
<400> 107
   aggtatttat cggcgcaagt tctgaaggcg tactgctgga aagag 45
<210> 108
   <211> 43
   <212> DNA
   <213> Artificial Sequence
<220>
   <223> F2 primer for ADH7
<400> 108
   ctcctctatg gtctagctgg caaagaccca caccaaggag ctg 43
<210> 109
   <211> 20
   <212> DNA
   <213> Artificial Sequence
<220>
   <223> R2 primer for ADH7
<400> 109
   caacgacacg accaacaatc 20
<210> 110
   <211> 20
   <212> DNA
   <213> Artificial Sequence
<220>
   <223> F1 primer for ADH8
<400> 110
   atcgcgccaa cttgtttaat 20
<210> 111
   <211> 45
   <212> DNA
   <213> Artificial Sequence
<220>
   <223> R1 primer for ADH8
<400> 111
   aggtatttat cggcgcaagt tctgacacct tctctcgtgg gatgt 45
<210> 112
   <211> 45
   <212> DNA
   <213> Artificial Sequence
<220>
   <223> F2 primer for ADH8
<400> 112
   ctcctctatg gtctagctgg caaagtgtgt tgagtctggc aaagc 45
<210> 113
   <211> 20
   <212> DNA
   <213> Artificial Sequence
<220>
   <223> R2 primer for ADH8
<400> 113
   tcaagtccat ggcatcaaac 20
<210> 114
   <211> 20
   <212> DNA
   <213> Artificial Sequence
<220>
   <223> F1 primer for FADH
<400> 114
   ccgaaggaaa gaccatcact 20
<210> 115
   <211> 45
   <212> DNA
   <213> Artificial Sequence
<220>
   <223> R1 primer for FADH
<400> 115
   ttgcacaagt aacgaacccg ccaatagaag gaagagcagc ccata 45
<210> 116
   <211> 45
   <212> DNA
   <213> Artificial Sequence
<220>
   <223> F2 primer for FADH
<400> 116
   ggagataata cggccttctt ccagggcttg ggcttacaag tttgg 45
<210> 117
   <211> 20
   <212> DNA
   <213> Artificial Sequence
<220>
   <223> R2 primer for FADH
<400> 117
   tcggtgaagg cagagttgat 20
<210> 118
   <211> 20
   <212> DNA
   <213> Artificial Sequence
<220>
   <223> F primer for HISG1
<400> 118
   cagaccggtt cagacaggat 20
<210> 119
   <211> 20
   <212> DNA
   <213> Artificial Sequence
<220>
   <223> R primer for HISG1
<400> 119
   ggaggtgcgg atatgaggta 20
<210> 120
   <211> 20
   <212> DNA
   <213> Artificial Sequence
<220>
   <223> F primer for HISG2
<400> 120
   aacgctacct cgaccagaaa 20
<210> 121
   <211> 20
   <212> DNA
   <213> Artificial Sequence
<220>
   <223> R primer for HISG2
<400> 121
   tcttctcgat cggcagtacc 20
<210> 122
   <211> 20
   <212> DNA
   <213> Artificial Sequence
<220>
   <223> F primer for glt2
<400> 122
   tcagaacttg cgccgataaa 20
<210> 123
   <211> 22
   <212> DNA
   <213> Artificial Sequence
<220>
   <223> R primer for glt2
<400> 123
   ctttgccagc tagaccatag ag 22
<210> 124
   <211> 19
   <212> DNA
   <213> Artificial Sequence
<220>
   <223> F primer for glt3
<400> 124
   attggcgggt tcgttactt 19
<210> 125
   <211> 22
   <212> DNA
   <213> Artificial Sequence
<220>
   <223> R primer for glt3
<400> 125
   cctggaagaa ggccgtatta tc 22
<210> 126
   <211> 23
   <212> DNA
   <213> Artificial Sequence
<220>
   <223> Ulura3 cs 2B primer for Bipartite
<400> 126
   atgccctcct acgaagctcg agc 23
<210> 127
   <211> 20
   <212> DNA
   <213> Artificial Sequence
<220>
   <223> Ylura3F primer for Bipartite
<400> 127
   ctcccaacga gaagctggcc 20